# EUROPÄISCHE PATENTSCHRIFT

(11) **EP 1 332 158 B1**
(45) Veröffentlichungstag und Bekanntmachung des Hinweises auf die Patenterteilung: **01.08.2007**
(21) Anmeldenummer: 01993614.5
(22) Anmeldetag: 08.11.2001
(51) Int. Cl.: C07K 14/705

(54) **PEPTIDE, DEREN HERSTELLUNG UND VERWENDUNG ZUR BINDUNG VON IMMUNGLOBULINEN**
PEPTIDES, THE PRODUCTION AND USE THEREOF FOR BINDING IMMUNOGLOBULINS
PEPTIDES, LEUR FABRICATION ET LEUR UTILISATION POUR LA LIAISON DES IMMUNOGLOBULINES

(30) Priorität: 08.11.2000 EP 00124418
(43) Veröffentlichungstag der Anmeldung: 06.08.2003
(73) Patentinhaber: Fresenius Medical Care Affina GmbH, 12489 Berlin (DE)
(72) Erfinder: EGNER, Ralf, 12051 Berlin (DE); WINKLER, Dirk, 10319 Berlin (DE); RÖNSPECK, Wolfgang, 10715 Berlin (DE); KUNZE, Rudolf, 17268 Stegelitz (DE)
(74) Vertreter: Meyers, Hans-Wilhelm
(86) Internationale Anmeldenummer: PCT/EP2001/012933
(87) Internationale Veröffentlichungsnummer: WO 2002/038592

(56) Entgegenhaltungen:
- EP-A- 1 086 955
- WO-A-01/45746
- KROOK M ET AL: "Novel peptides binding to the Fc-portion of immunoglobulins obtained from a combinatorial phage display peptide library" JOURNAL OF IMMUNOLOGICAL METHODS, ELSEVIER SCIENCE PUBLISHERS B.V.,AMSTERDAM, NL, Bd. 221, Nr. 1-2, Dezember 1998 (1998-12), Seiten 151-157, XP004153568 ISSN: 0022-1759
- DELANO WARREN L ET AL: "Convergent solutions to binding at a protein-protein interface." SCIENCE (WASHINGTON D C), Bd. 287, Nr. 5456, 18. Februar 2000 (2000-02-18), Seiten 1279-1283, XP002211313 ISSN: 0036-8075

### SEQUENZPROTOKOLL

<110> Affina Immuntechnik GmbH
<120> Peptide, deren Herstellung und Verwendung zur Bindung von Immunglobulinen
<130> 012827wo
<140> PCT/EP01/12933
   <141> 2001-11-08
<150> EP00124418.5
   <151> 2000-11-08
<160> 88
<170> PatentIn Ver. 2.1
<210> 1
   <211> 13
   <212> PRT
   <213> Künstliche Sequenz
<220>
   <223> Beschreibung der künstlichen Sequenz: synthetisches Peptid mit Affinität zu Immunglobulinen
<400> 1
   Ala Cys Ala Trp His Leu Gly Lys Leu Val Trp Cys Thr
    1               5                  10
<210> 2
   <211> 13
   <212> PRT
   <213> Künstliche Sequenz
<220>
   <223> Beschreibung der künstlichen Sequenz: synthetisches Peptid mit Affinität zu Immunglobulinen
<400> 2
   Glu Cys Ala Trp His Leu Gly Lys Leu Val Trp Cys Thr
    1               5                  10
<210> 3
   <211> 13
   <212> PRT
   <213> Künstliche Sequenz
<220> <223> Beschreibung der künstlichen Sequenz: synthetisches Peptid mit Affinität zu Immunglobulinen
<400> 3
   Gly Cys Ala Trp His Leu Gly Lys Leu Val Trp Cys Thr
   1 5 10
<210> 4
   <211> 12
   <212> PRT
   <213> Künstliche Sequenz
<220>
   <223> Beschreibung der künstlichen Sequenz: synthetisches Peptid mit Affinität zu Immunglobulinen
<400> 4
   Cys Ala Trp His Leu Gly Lys Leu Val Trp Cys Thr
   1 5 10
<210> 5
   <211> 13
   <212> PRT
   <213> Künstliche Sequenz
<220>
   <223> Beschreibung der künstlichen Sequenz: synthetisches Peptid mit Affinität zu Immunglobulinen
<400> 5
   Asp Ser Ala Trp His Leu Gly Lys Leu Val Trp Cys Thr
   1 5 10
<210> 6
   <211> 13
   <212> PRT
   <213> Künstliche Sequenz
<220>
   <223> Beschreibung der künstlichen Sequenz: synthetisches Peptid mit Affinität zu Immunglobulinen
<400> 6
   Asp Cys Ser Trp His Leu Gly Lys Leu Val Trp Cys Thr
   1 5 10
<210> 7
   <211> 13
   <212> PRT
   <213> Künstliche Sequenz
<220>
   <223> Beschreibung der künstlichen Sequenz: synthetisches Peptid mit Affinität zu Immunglobulinen
<400> 7
   Asp Cys Thr Trp His Leu Gly Lys Leu Val Trp Cys Thr
   1 5 10
<210> 8
   <211> 13
   <212> PRT
   <213> Künstliche Sequenz
<220>
   <223> Beschreibung der künstlichen Sequenz: synthetisches Peptid mit Affinität zu Immunglobulinen
<400> 8
   Asp Cys Ala Glu His Leu Gly Lys Leu Val Trp Cys Thr
   1 5 10
<210> 9
   <211> 13
   <212> PRT
   <213> Künstliche Sequenz
<220>
   <223> Beschreibung der künstlichen Sequenz: synthetisches Peptid mit Affinität zu Immunglobulinen
<400> 9
   Asp Cys Ala Phe His Leu Gly Lys Leu Val Trp Cys Thr
   1 5 10
<210> 10
   <211> 13
   <212> PRT
   <213> Künstliche Sequenz
<220>
   <223> Beschreibung der künstlichen Sequenz: synthetisches Peptid mit Affinität zu Immunglobulinen
<400> 10
   Asp Cys Ala His His Leu Gly Lys Leu Val Trp Cys Thr
   1 5 10
<210> 11
   <211> 13
   <212> PRT
   <213> Künstliche Sequenz
<220>
   <223> Beschreibung der künstlichen Sequenz: synthetisches Peptid mit Affinität zu. Immunglobulinen
<400> 11
   Asp Cys Ala Lys His Leu Gly Lys Leu Val Trp Cys Thr
    1               5                  10
<210> 12
   <211> 13
   <212> PRT
   <213> Künstliche Sequenz
<220>
   <223> Beschreibung der künstlichen Sequenz: synthetisches Peptid mit Affinität zu Immunglobulinen
<400> 12
   Asp Cys Ala Met His Leu Gly Lys Leu Val Trp Cys Thr
    1               5                  10
<210> 13
   <211> 13
   <212> PRT
   <213> Künstliche Sequenz
<220>
   <223> Beschreibung der künstlichen Sequenz: synthetisches Peptid mit Affinität zu Immunglobulinen
<400> 13
   Asp Cys Ala Arg His Leu Gly Lys Leu Val Trp Cys Thr
    1               5                  10
<210> 14
   <211> 13
   <212> PRT
   <213> Künstliche Sequenz
<220>
   <223> Beschreibung der künstlichen Sequenz: synthetisches Peptid mit Affinität zu Immunglobulinen
<400> 14
   Asp Cys Ala Ser His Leu Gly Lys Leu Val Trp Cys Thr
    1               5                  10
<210> 15
   <211> 13
   <212> PRT
   <213> Künstliche Sequenz
<220>
   <223> Beschreibung der künstlichen Sequenz: synthetisches Peptid mit Affinität zu Immunglobulinen
<400> 15
   Asp Cys Ala Thr His Leu Gly Lys Leu Val Trp Cys Thr
   1 5 10
<210> 16
   <211> 13
   <212> PRT
   <213> Künstliche Sequenz
<220>
   <223> Beschreibung der künstlichen Sequenz: synthetisches Peptid mit Affinität zu Immunglobulinen
<400> 16
   Asp Cys Ala Val His Leu Gly Lys Leu Val Trp Cys Thr
   1 5 10
<210> 17
   <211> 13
   <212> PRT
   <213> Künstliche Sequenz
<220>
   <223> Beschreibung der künstlichen Sequenz: synthetisches Peptid mit Affinität zu Immunglobulinen
<400> 17
   Asp Cys Ala Trp His Leu Gly Lys Leu Val Trp Cys Thr
   1 5 10
<210> 18
   <211> 13
   <212> PRT
   <213> Künstliche Sequenz
<220>
   <223> Beschreibung der künstlichen Sequenz: synthetisches Peptid mit Affinität zu Immunglobulinen
<400> 18
   Asp Cys Ala Tyr His Leu Gly Lys Leu Val Trp Cys Thr
   1 5 10
<210> 19
   <211> 13
   <212> PRT
   <213> Künstliche Sequenz
<220>
   <223> Beschreibung der künstlichen Sequenz: synthetisches Peptid mit Affinität zu Immunglobulinen
<400> 19
   Asp Cys Ala Trp His Asp Gly Lys Leu Val Trp Cys Thr
   1 5 10
<210> 20
   <211> 13
   <212> PRT
   <213> Künstliche Sequenz
<220>
   <223> Beschreibung der künstlichen Sequenz: synthetisches Peptid mit Affinität zu Immunglobulinen
<400> 20
   Asp Cys Ala Trp His Glu Gly Lys Leu Val Trp Cys Thr
   1 5 10
<210> 21
   <211> 13
   <212> PRT
   <213> Künstliche Sequenz
<220>
   <223> Beschreibung der künstlichen Sequenz: synthetisches Peptid mit Affinität zu Immunglobulinen
<400> 21
   Asp Cys Ala Trp His Gly Gly Lys Leu Val Trp Cys Thr
   1 5 10
<210> 22
   <211> 13
   <212> PRT
   <213> Künstliche Sequenz
<220>
   <223> Beschreibung der künstlichen Sequenz: synthetisches Peptid mit Affinität zu Immunglobulinen
<400> 22
   Asp Cys Ala Trp His His Gly Lys Leu Val Trp Cys Thr
   1 5 10
<210> 23
   <211> 13
   <212> PRT
   <213> Künstliche Sequenz
<220>
   <223> Beschreibung der künstlichen Sequenz: synthetisches Peptid mit Affinität zu Immunglobulinen
<400> 23
   Asp Cys Ala Trp His Lys Gly Lys Leu Val Trp Cys Thr
   1 5 10
<210> 24
   <211> 13
   <212> PRT
   <213> Künstliche Sequenz
<220>
   <223> Beschreibung der künstlichen Sequenz: synthetisches Peptid mit Affinität zu Immunglobulinen
<400> 24
   Asp Cys Ala Trp His Met Gly Lys Leu Val Trp Cys Thr
   1 5 10
<210> 25
   <211> 13
   <212> PRT
   <213> Künstliche Sequenz
<220>
   <223> Beschreibung der künstlichen Sequenz: synthetisches Peptid mit Affinität zu Immunglobulinen
<400> 25
   Asp Cys Ala Trp His Asn Gly Lys Leu Val Trp Cys Thr
   1 5 10
<210> 26
   <211> 13
   <212> PRT
   <213> Künstliche Sequenz
<220>
   <223> Beschreibung der künstlichen Sequenz: synthetisches Peptid mit Affinität zu Immunglobulinen
<400> 26
   Asp Cys Ala Trp His Gln Gly Lys Leu Val Trp Cys Thr
   1 5 10
<210> 27
   <211> 13
   <212> PRT
   <213> Künstliche Sequenz
<220>
   <223> Beschreibung der künstlichen Sequenz: synthetisches Peptid mit Affinität zu Immunglobulinen
<400> 27
   Asp Cys Ala Trp His Arg Gly Lys Leu Val Trp Cys Thr
   1 5 10
<210> 28
   <211> 13
   <212> PRT
   <213> Künstliche Sequenz
<220>
   <223> Beschreibung der künstlichen Sequenz: synthetisches Peptid mit Affinität zu Immunglobulinen
<400> 28
   Asp Cys Ala Trp His Leu Asp Lys Leu Val Trp Cys Thr
   1 5 10
<210> 29
   <211> 13
   <212> PRT
   <213> Künstliche Sequenz
<220>
   <223> Beschreibung der künstlichen Sequenz: synthetisches Peptid mit Affinität zu Immunglobulinen
<400> 29
   Asp Cys Ala Trp His Leu Gly Asp Leu Val Trp Cys Thr
   1 5 10
<210> 30
   <211> 13
   <212> PRT
   <213> Künstliche Sequenz
<220>
   <223> Beschreibung der künstlichen Sequenz: synthetisches Peptid mit Affinität zu Immunglobulinen
<400> 30
   Asp Cys Ala Trp His Leu Gly His Leu Val Trp Cys Thr
   1 5 10
<210> 31
   <211> 13
   <212> PRT
   <213> Künstliche Sequenz
<220>
   <223> Beschreibung der künstlichen Sequenz: synthetisches Peptid mit Affinität zu Immunglobulinen
<400> 31
   Asp Cys Ala Trp His Leu Gly Lys Leu Val Trp Cys Thr
   1 5 10
<210> 32
   <211> 13
   <212> PRT
   <213> Künstliche Sequenz
<220>
   <223> Beschreibung der künstlichen Sequenz: synthetisches Peptid mit Affinität zu Immunglobulinen
<400> 32
   Asp Cys Ala Trp His Leu Gly Met Leu Val Trp Cys Thr
   1 5 10
<210> 33
   <211> 13
   <212> PRT
   <213> Künstliche Sequenz
<220>
   <223> Beschreibung der künstlichen Sequenz: synthetisches Peptid mit Affinität zu Immunglobulinen
<400> 33
   Asp Cys Ala Trp His Leu Gly Asn Leu Val Trp Cys Thr
   1 5 10
<210> 34
   <211> 13
   <212> PRT
   <213> Künstliche Sequenz
<220>
   <223> Beschreibung der künstlichen Sequenz: synthetisches Peptid mit Affinität zu Immunglobulinen
<400> 34
   Asp Cys Ala Trp His Leu Gly Gln Leu Val Trp Cys Thr
    1               5                  10
<210> 35
   <211> 13
   <212> PRT
   <213> Künstliche Sequenz
<220>
   <223> Beschreibung der künstlichen Sequenz: synthetisches Peptid mit Affinität zu Immunglobulinen
<400> 35
   Asp Cys Ala Trp His Leu Gly Arg Leu Val Trp Cys Thr
    1               5                  10
<210> 36
   <211> 13
   <212> PRT
   <213> Künstliche Sequenz
<220>
   <223> Beschreibung der künstlichen Sequenz: synthetisches Peptid mit Affinität zu Immunglobulinen
<400> 36
   Asp Cys Ala Trp His Leu Gly Lys Lys Val Trp Cys Thr
    1               5                  10
<210> 37
   <211> 13
   <212> PRT
   <213> Künstliche Sequenz
<220>
   <223> Beschreibung der künstlichen Sequenz: synthetisches Peptid mit Affinität zu Immunglobulinen
<400> 37
   Asp Cys Ala Trp His Leu Gly Lys Arg Val Trp Cys Thr
    1               5                  10
<210> 38
   <211> 13
   <212> PRT
   <213> Künstliche Sequenz
<220>
<223> Beschreibung der künstlichen Sequenz: synthetisches Peptid mit Affinität zu Immunglobulinen
<400> 38
   Asp Cys Ala Trp His Leu Gly Lys Leu Val Trp Ser Thr
   1 5 10
<210> 39
   <211> 13
   <212> PRT
   <213> Künstliche Sequenz
<220>
   <223> Beschreibung der künstlichen Sequenz: synthetisches Peptid mit Affinität zu Immunglobulinen
<400> 39
   Asp Cys Ala Trp His Leu Gly Lys Leu Val Trp Cys Asp
   1 5 10
<210> 40
   <211> 13
   <212> PRT
   <213> Künstliche Sequenz
<220>
   <223> Beschreibung der künstlichen Sequenz: synthetisches Peptid mit Affinität zu Immunglobulinen
<400> 40
   Asp Cys Ala Trp His Leu Gly Lys Leu Val Trp Cys Glu
   1 5 10
<210> 41
   <211> 13
   <212> PRT
   <213> Künstliche Sequenz
<220>
   <223> Beschreibung der künstlichen Sequenz: synthetisches Peptid mit Affinität zu Immunglobulinen
<400> 41
   Asp Cys Ala Trp His Leu Gly Lys Leu Val Trp Cys Lys
   1 5 10
<210> 42
   <211> 13
   <212> PRT
   <213> Künstliche Sequenz
<220>
   <223> Beschreibung der künstlichen Sequenz: synthetisches Peptid mit Affinität zu Immunglobulinen
<400> 42
   Asp Cys Ala Trp His Leu Gly Lys Leu Val Trp Cys Gln
   1 5 10
<210> 43
   <211> 13
   <212> PRT
   <213> Künstliche Sequenz
<220>
   <223> Beschreibung der künstlichen Sequenz: synthetisches Peptid mit Affinität zu Immunglobulinen
<400> 43
   Asp Cys Ala Trp His Leu Gly Lys Leu Val Trp Cys Arg
   1 5 10
<210> 44
   <211> 13
   <212> PRT
   <213> Künstliche Sequenz
<220>
   <223> Beschreibung der künstlichen Sequenz: synthetisches Peptid mit Affinität zu Immunglobulinen
<400> 44
   Asp Cys Ala Trp His Leu Gly Lys Leu Val Trp Cys Ser
   1 5 10
<210> 45
   <211> 12
   <212> PRT
   <213> Künstliche Sequenz
<220>
   <223> Beschreibung der künstlichen Sequenz: synthetisches Peptid mit Affinität zu Immunglobulinen
<400> 45
   Asp Cys Ala Trp His Leu Gly Lys Leu Val Trp Cys
   1 5 10
<210> 46
   <211> 12
   <212> PRT
   <213> Künstliche Sequenz
<220>
   <223> Beschreibung der künstlichen Sequenz: synthetisches Peptid mit Affinität zu Immunglobulinen
<400> 46
   Cys Ala Trp His Leu Gly Lys Leu Val Trp Cys Thr
   1 5 10
<210> 47
   <211> 12
   <212> PRT
   <213> Künstliche Sequenz
<220>
   <223> Beschreibung der künstlichen Sequenz: synthetisches Peptid mit Affinität zu Immunglobulinen
<400> 47
   Asp Cys Ala Trp His Leu Gly Lys Leu Val Trp Cys
   1 5 10
<210> 48
   <211> 11
   <212> PRT
   <213> Künstliche Sequenz
<220>
   <223> Beschreibung der künstlichen Sequenz: synthetisches Peptid mit Affinität zu Immunglobulinen
<400> 48
   Cys Ala Trp His Leu Gly Lys Leu Val Trp Cys
   1 5 10
<210> 49
   <211> 13
   <212> PRT
   <213> Künstliche Sequenz
<220>
   <223> Beschreibung der künstlichen Sequenz: synthetisches Peptid mit Affinität zu Immunglobulinen
<400> 49
   Asp Cys Ser Trp His Leu Gly Lys Leu Val Trp Cys Thr
   1 5 10
<210> 50
   <211> 13
   <212> PRT
   <213> Künstliche Sequenz
<220>
   <223> Beschreibung der künstlichen Sequenz: synthetisches Peptid mit Affinität zu Immunglobulinen
<400> 50
   Asp Cys Ala Trp His His Gly Lys Leu Val Trp Cys Thr
   1 5 10
<210> 51
   <211> 13
   <212> PRT
   <213> Künstliche Sequenz
<220>
   <223> Beschreibung der künstlichen Sequenz: synthetisches Peptid mit Affinität zu Immunglobulinen
<400> 51
   Asp Cys Ala Trp His Gln Gly Lys Leu Val Trp Cys Thr
   1 5 10
<210> 52
   <211> 13
   <212> PRT
   <213> Künstliche Sequenz
<220>
   <223> Beschreibung der künstlichen Sequenz: synthetisches Peptid mit Affinität zu Immunglobulinen
<400> 52
   Asp Cys Ala Trp His Gly Gly Lys Leu Val Trp Cys Thr
   1 5 10
<210> 53
   <211> 13
   <212> PRT
   <213> Künstliche Sequenz
<220>
   <223> Beschreibung der künstlichen Sequenz: synthetisches Peptid mit Affinität zu Immunglobulinen
<400> 53
   Asp Cys Ala Trp His Gly Gly Lys Leu Val Trp Cys Thr
   1 5 10
<210> 54
   <211> 13
   <212> PRT
   <213> Künstliche Sequenz
<220>
   <223> Beschreibung der künstlichen Sequenz: synthetisches Peptid mit Affinität zu Immunglobulinen
<400> 54
   Asp Cys Ala Trp His Gly Gly His Leu Val Trp Cys Thr
   1 5 10
<210> 55
   <211> 13
   <212> PRT
   <213> Künstliche Sequenz
<220>
   <223> Beschreibung der künstlichen Sequenz: synthetisches Peptid mit Affinität zu Immunglobulinen
<400> 55
   Asp Cys Ala Trp His Gly Gly Lys Leu Val Trp Cys Glu
   1 5 10
<210> 56
   <211> 13
   <212> PRT
   <213> Künstliche Sequenz
<220>
   <223> Beschreibung der künstlichen Sequenz: synthetisches Peptid mit Affinität zu Immunglobulinen
<400> 56
   Asp Cys Ala Trp His Leu Gly Lys Leu Val Trp Cys Thr
   1 5 10
<210> 57
   <211> 13
   <212> PRT
   <213> Künstliche Sequenz
<220>
   <223> Beschreibung der künstlichen Sequenz: synthetisches Peptid mit Affinität zu Immunglobulinen
<400> 57
   Asp Cys Ala Trp His Gln Gly Lys Leu Val Trp Cys Thr
    1               5                   10
<210> 58
   <211> 13
   <212> PRT
   <213> Künstliche Sequenz
<220>
   <223> Beschreibung der künstlichen Sequenz: synthetisches Peptid mit Affinität zu Immunglobulinen
<400> 58
   Asp Cys Ala Tyr His Leu Gly Lys Leu Val Trp Cys Thr
    1               5                   10
<210> 59
   <211> 13
   <212> PRT
   <213> Künstliche Sequenz
<220>
   <223> Beschreibung der künstlichen Sequenz: synthetisches Peptid mit Affinität zu Immunglobulinen
<400> 59
   Asp Cys Ser Trp His Leu Gly Lys Leu Val Trp Cys Thr
    1               5                   10
<210> 60
   <211> 13
   <212> PRT
   <213> Künstliche Sequenz
<220>
   <223> Beschreibung der künstlichen Sequenz: synthetisches Peptid mit Affinität zu Immunglobulinen
<400> 60
   Asp Cys Ala Tyr His Gln Gly Lys Leu Val Trp Cys Thr
    1               5                   10
<210> 61
   <211> 13
   <212> PRT
   <213> Künstliche Sequenz
<220>
   <223> Beschreibung der künstlichen sequenz: synthetisches Peptid mit Affinität zu Immunglobulinen
<400> 61
   Asp Cys Ser Trp His Gln Gly Lys Leu Val Trp Cys Thr
   1 5 10
<210> 62
   <211> 13
   <212> PRT
   <213> Künstliche Sequenz
<220>
   <223> Beschreibung der künstlichen Sequenz: synthetisches Peptid mit Affinität zu Immunglobulinen
<400> 62
   Asp Cys Ser Tyr His Leu Gly Lys Leu Val Trp Cys Thr
   1 5 10
<210> 63
   <211> 13
   <212> PRT
   <213> Künstliche Sequenz
<220>
   <223> Beschreibung der künstlichen Sequenz: synthetisches Peptid mit Affinität zu Immunglobulinen
<400> 63
   Asp Cys Ser Tyr His Gln Gly Lys Leu Val Trp Cys Thr
   1 5 10
<210> 64
   <211> 12
   <212> PRT
   <213> Künstliche Sequenz
<220>
   <223> Beschreibung der künstlichen Sequenz: synthetisches Peptid mit Affinität zu Immunglobulinen
<400> 64
   Cys Ala Trp His Leu Gly Lys Leu Val Trp Cys Thr
   1 5 10
<210> 65
   <211> 12
   <212> PRT
   <213> Künstliche Sequenz
<220>
   <223> Beschreibung der künstlichen Sequenz: synthetisches Peptid mit Affinität zu Immunglobulinen
<400> 65
   Cys Ala Trp His Gln Gly Lys Leu Val Trp Cys Thr
    1               5                   10
<210> 66
   <211> 12
   <212> PRT
   <213> Künstliche Sequenz
<220>
   <223> Beschreibung der künstlichen Sequenz: synthetisches Peptid mit Affinität zu Immunglobulinen
<400> 66
   Cys Ala Tyr His Leu Gly Lys Leu Val Trp Cys Thr
    1               5                   10
<210> 67
   <211> 12
   <212> PRT
   <213> Künstliche Sequenz
<220>
   <223> Beschreibung der künstlichen Sequenz: synthetisches Peptid mit Affinität zu Immunglobulinen
<400> 67
   Cys Ser Trp His Leu Gly Lys Leu Val Trp Cys Thr
    1               5                   10
<210> 68
   <211> 12
   <212> PRT
   <213> Künstliche Sequenz
<220>
   <223> Beschreibung der künstlichen Sequenz: synthetisches Peptid mit Affinität zu Immunglobulinen
<400> 68
   Cys Ala Tyr His Gln Gly Lys Leu Val Trp Cys Thr
    1               5                   10
<210> 69
   <211> 12
   <212> PRT
   <213> Künstliche Sequenz
<220>
   <223> Beschreibung der künstlichen Sequenz: synthetisches Peptid mit Affinität zu Immunglobulinen
<400> 69
   Cys Ser Trp His Gln Gly Lys Leu Val Trp Cys Thr
    1               5                   10
<210> 70
   <211> 12
   <212> PRT
   <213> Künstliche Sequenz
<220>
   <223> Beschreibung der künstlichen Sequenz: synthetisches Peptid mit Affinität zu Immunglobulinen
<400> 70
   Cys Ser Tyr His Leu Gly Lys Leu Val Trp Cys Thr
    1               5                   10
<210> 71
   <211> 12
   <212> PRT
   <213> Künstliche Sequenz
<220>
   <223> Beschreibung der künstlichen Sequenz: synthetisches Peptid mit Affinität zu Immunglobulinen
<400> 71
   Cys Ser Tyr His Gln Gly Lys Leu Val Trp Cys Thr
    1               5                   10
<210> 72
   <211> 12
   <212> PRT
   <213> Künstliche Sequenz
<220>
   <223> Beschreibung der künstlichen Sequenz: synthetisches Peptid mit Affinität zu Immunglobulinen
<400> 72
   Asp Cys Ala Trp His Leu Gly Lys Leu Val Trp Cys
    1               5                   10
<210> 73
   <211> 12
   <212> PRT
   <213> Künstliche Sequenz
<220>
   <223> Beschreibung der künstlichen Sequenz: synthetisches Peptid mit Affinität zu Immunglobulinen
<400> 73
   Asp Cys Ala Trp His Gln Gly Lys Leu Val Trp Cys
   1 5 10
<210> 74
   <211> 12
   <212> PRT
   <213> Künstliche Sequenz
<220>
   <223> Beschreibung der künstlichen Sequenz: synthetisches Peptid mit Affinität zu Immunglobulinen
<400> 74
   Asp Cys Ala Tyr His Leu Gly Lys Leu Val Trp Cys
   1 5 10
<210> 75
   <211> 12
   <212> PRT
   <213> Künstliche Sequenz
<220>
   <223> Beschreibung der künstlichen Sequenz: synthetisches Peptid mit Affinität zu Immunglobulinen
<400> 75
   Asp Cys Ser Trp His Leu Gly Lys Leu Val Trp Cys
   1 5 10
<210> 76
   <211> 12
   <212> PRT
   <213> Künstliche Sequenz
<220>
   <223> Beschreibung der künstlichen Sequenz: synthetisches Peptid mit Affinität zu Immunglobulinen
<400> 76
   Asp Cys Ala Tyr His Gln Gly Lys Leu Val Trp Cys
   1 5 10
<210> 77
   <211> 12
   <212> PRT
   <213> Künstliche Sequenz
<220>
   <223> Beschreibung der künstlichen Sequenz: synthetisches Peptid mit Affinität zu Immunglobulinen
<400> 77
   Asp Cys Ser Trp His Gln Gly Lys Leu Val Trp Cys
   1 5 10
<210> 78
   <211> 12
   <212> PRT
   <213> Künstliche Sequenz
<220>
   <223> Beschreibung der künstlichen Sequenz: synthetisches Peptid mit Affinität zu Immunglobulinen
<400> 78
   Asp Cys Ser Tyr His Leu Gly Lys Leu Val Trp Cys
   1 5 10
<210> 79
   <211> 12
   <212> PRT
   <213> Künstliche Sequenz
<220>
   <223> Beschreibung der künstlichen Sequenz: synthetisches Peptid mit Affinität zu Immunglobulinen
<400> 79
   Asp Cys Ser Tyr His Gln Gly Lys Leu Val Trp Cys
   1 5 10
<210> 80
   <211> 11
   <212> PRT
   <213> Künstliche Sequenz
<220>
   <223> Beschreibung der künstlichen Sequenz: synthetisches Peptid mit Affinität zu Immunglobulinen
<400> 80
   Cys Ala Trp His Leu Gly Lys Leu Val Trp Cys
   1 5 10
<210> 81
   <211> 11
   <212> PRT
   <213> Künstliche Sequenz
<220>
   <223> Beschreibung der künstlichen Sequenz: synthetisches Peptid mit Affinität zu Immunglobulinen
<400> 81
   Cys Ala Trp His Gln Gly Lys Leu Val Trp Cys
   1 5 10
<210> 82
   <211> 11
   <212> PRT
   <213> Künstliche Sequenz
<220>
   <223> Beschreibung der künstlichen Sequenz: synthetisches Peptid mit Affinität zu Immunglobulinen
<400> 82
   Cys Ala Tyr His Leu Gly Lys Leu Val Trp Cys
   1 5 10
<210> 83
   <211> 11
   <212> PRT
   <213> Künstliche Sequenz
<220>
   <223> Beschreibung der künstlichen Sequenz: synthetisches Peptid mit Affinität zu Immunglobulinen
<400> 83
   Cys Ser Trp His Leu Gly Lys Leu Val Trp Cys
   1 5 10
<210> 84
   <211> 11
   <212> PRT
   <213> Künstliche Sequenz
<220>
   <223> Beschreibung der künstlichen Sequenz: synthetisches Peptid mit Affinität zu Immunglobulinen
<400> 84
   Cys Ala Tyr His Gln Gly Lys Leu Val Trp Cys
   1 5 10
<210> 85
   <211> 11
   <212> PRT
   <213> Künstliche Sequenz
<220>
   <223> Beschreibung der künstlichen Sequenz: synthetisches Peptid mit Affinität zu Immunglobulinen
<400> 85
   Cys Ser Trp His Gln Gly Lys Leu Val Trp Cys
   1 5 10
<210> 86
   <211> 11
   <212> PRT
   <213> Künstliche Sequenz
<220>
   <223> Beschreibung der künstlichen Sequenz: synthetisches Peptid mit Affinität zu Immunglobulinen
<400> 86
   Cys Ser Tyr His Leu Gly Lys Leu Val Trp Cys
   1 5 10
<210> 87
   <211> 11
   <212> PRT
   <213> Künstliche Sequenz
<220>
   <223> Beschreibung der künstlichen Sequenz: synthetisches Peptid mit Affinität zu Immunglobulinen
<400> 87
   Cys Ser Tyr His Gln Gly Lys Leu Val Trp Cys
   1 5 10
<210> 88
   <211> 13
   <212> PRT
   <213> Künstliche Sequenz
<220>
   <223> Beschreibung der künstlichen Sequenz: synthetisches Peptid mit Affinität zu Immunglobulinen
<400> 88
   Asp Cys Ala Trp His Leu Gly Glu Leu Val Trp Cys Thr
    1               5                   10

## Beschreibung

Gegenstand der vorliegenden Erfindung sind Peptide mit Affinität zu Immunglobulinen, feste Phasen an denen die erfindungsgemäßen Peptide gebunden sind, ein Verfahren zur Adsorption von Immunoglobulinen, eine Vorrichtung zur Durchführung des Verfahrens sowie Verwendungen der erfindungsgemäßen Peptide.

Immunglobuline sind bei allen Vertebraten vorkommende Proteinmoleküle, die der körpereigenen spezifischen Immunabwehr dienen, indem sie an Antigene, mikrobielle sowie andere körperfremde Strukturen, binden und diese neutralisieren. Antigentragende Strukturen wie z.B. in den Körper eingedrungene Bakterien oder Parasiten sowie virusinfizierte Körperzellen werden durch gebundene Immunglobuline für eine Vernichtung durch entsprechend spezialisierte Zellen oder Molekülsysteme, wie z. B. das Komplementsystem, kenntlich gemacht. Immunglobuline werden je nach der Struktur ihrer molekularen Untereinheiten verschiedenen Immunglobulinklassen zugeordnet, die sich auch hinsichtlich ihrer biologischen Aktivität, wie z.B. der Fähigkeit zur Komplementaktivierung oder Schleimhautgängigkeit unterscheiden. Allgemein unterscheiden sich alle Immunglobuline, unabhängig von der Immunglobulinklasse, in ihrer variablen, für das jeweilige Antigen spezifischen Bindungsstelle. Alle Immunglobuline haben gemeinsame Strukturmerkmale, über die sie auch gemeinsam gebunden werden können, wobei hierfür die "Fc-Region" genannte molekulare Untereinheit bevorzugt wird (Davies and Metzger (1983); Alt et al. (1987)).

In der Natur vorkommende Immunglobulin-bindende Moleküle sind vor allem Oberflächenrezeptoren von Körperzellen, welche die Fc-Region der verschiedenen Immunglobuline erkennen und an diese binden. Nachdem sie Immunglobuline gebunden haben, werden über die Rezeptoren, in Abhängigkeit von der gebundenen Immunglobulinklasse und dem Zelltyp unterschiedliche Aktivierungsvorgänge ausgelöst (McKenzie and Schreiber (1994); Makiya and Stigbrand (1992); Sarfati et al. (1992); Capron et al. (1992); Shen (1992); Sandor et al. (1992)).

Neben Oberflächenrezeptoren binden auch Moleküle des Komplementsystems an die Fc-Region von Immunglobulinen. Das Komplementsystem stellt eine System von aufeinander abgestimmten Proteinen dar, welches der Zerstörung der antigentragenden Zielstruktur dient und zwei unterschiedliche Aktivierungswege aufweist. Bei dem einen - dem klassische Weg - erfolgt die Aktivierung über die Bindung der Komplementkomponente C1 bzw. deren Untereinheit C1q an die Fc-Region der antigengebundenen Immunglobuline (Miletic and Frank (1995)).

Auch einige Bakterien haben Proteine entwickelt, die in der Lage sind, Immunglobuline über die Fc-Region zu binden. Dazu gehören das Protein A der Staphylokokken und das Protein G der Streptokokken. Diese Proteine und deren Abkömmlinge sind interessante Arbeitsmittel in der immunologischen Forschung, beispielsweise beim Studium der Rezeptor-Immunglobulin-Interaktion und finden gekoppelt an eine Trägermatrix breiten Einsatz in der affinitätschromatographischen Reinigung von Immunglobulinen (Stahl et al. (1993)).

Ausgehend von dieser Anwendung wurde die therapeutische Immunapherese zur Behandlung von Autoimmunerkrankungen etabliert. Bei diesem Verfahren werden auf der Basis von matrixgekoppeltem Protein A Immunglobuline aus humanem Plasma entfernt und damit auch die Konzentration von pathogenen Autoantikörpern abgesenkt (Belak et al. (1994)).

Auf dem Markt befinden sich derzeit zwei Protein A-Produkte, die sich hinsichtlich der verwendeten Adsorbermatrix unterscheiden. Zum einen wird Protein A immobilisiert an Agarose (Sepharose) als Trägermatrix verwendet (Immunosorba, Fresenius HemoCare AG/Deutschland) (Samuelsson (1998)), zum anderen findet Kieselgel als Trägermatrix Anwendung (Prosorba, Cypress Bioscience Inc./USA). Die Anwendung der Immunapherese mit Protein A zur Entfernung der Immunglobuline wurde bei verschiedenen Autoimmunerkrankungen, beispielsweise der Rheumatoiden Arthritis erfolgreich eingesetzt (Felson et al. (1999)).

Weiterhin können nach Immunisierung mit Immunglobulinen einer bestimmten Spezies in einer anderen Spezies Antikörper induziert werden, die dann in der Lage sind die Immunglobuline der ersten Spezies zu binden. Hierauf beruht ein Produkt zur therapeutischen Immunapherese, welches matrixgekoppelte Antikörper vom Schafen verwendet, die aus dem Serum der Tiere nach Immunisierung mit menschlichen Immunglobulinen gewonnen werden. Auch dieses System (Ig-Therasorb, PlasmaSelect AG/Deutschland) findet Einsatz bei der Gesamtimmunglobulinentfernung und dient somit auch der therapeutischen Entfernung von pathogenen Antikörpern (Koll (1998)), beispielsweise von Faktor VIII- und Faktor IX-Inhibitoren (Knobl and Derfler (1999)).

Auch matrix-immobilisierte Aminosäuren werden für die therapeutische Immunapherese eingesetzt. Hierzu kommen Adsorber auf Phenylalanin- (IM-PH350, Asahi Medical Co. Ltd. /Japan) und Tryptophanbasis (IM-TR350, Asahi/Japan) zum Einsatz (Jimenez et al. (1993); Fadul et al. (1996)). Die Bindungsfähigkeit dieser Adsorber ist jedoch im Gegensatz zu den vorgenannten Adsorbersystemen nicht auf Immunglobuline beschränkt, sondern es werden auch andere Proteine, wie das für die Gerinnung notwendige Fibrinogen, aus dem Blutplasma entfernt.

Ein vollkommen anderes Bindungsprinzip wird bei der Verwendung von Peptiden als Antigen oder Antigenmimetikum zur Entfernung pathogener Autoantikörper angewandt. Beispielsweise wird der Adsorber MG-50 (Kuraray Co. Ltd./Japan) zur Eliminierung von Autoantikörpern gegen den Acetylcholinrezeptor bei der Behandlung von Myasthenia gravis angewendet (Takamori and Ide (1996)). Ein weiteres Beispiel ist die spezifische, immunapheretische Eliminierung von Autoantikörpern gegen den β1-adrenergen Rezeptor (EP 99 118 631), Affina Immuntechnik GmbH/Deutschland) bei der Therapie der Dilatativen Cardiomyopathie (DCM). D.h. in den genannten Beispielen werden nur die Immunglobuline entfernt, die gegen das jeweilige Peptidantigen gerichtet sind, während alle anderen Immunglobuline nicht gebunden werden. Die Verwendung von Peptiden zur Bindung ausgewählter Immunglobuline über ihre Antigenbindungsstelle ist Stand der Technik.

Interessanterweise und im Gegensatz zu den oben beschriebenen Peptiden sind in der Literatur auch Peptide beschrieben worden, die nicht über die Antigenbindungsstelle eines Immunglobulins gebunden werden, sondern an die Fc-Region von Immunglobulinen binden. So wurde ein Protein A-mimetisches Peptid beschrieben, das sich zur technischen Reinigung von Immunglobulinen eignet (Fassina et al. (1996) ; Fassina et al. (1998)). Weiterhin wurde ein Peptid beschrieben, das in gepufferter Lösung einen monoklonalen IgG-Antikörper im Bereich der Protein A-Bindungsstelle des Immunglobulins bindet (DeLano et al. (2000)) sowie WO-A-01/45746. Andere ebenfalls in der Fc-Region bindende Peptide, bestehend aus 10 Aminosäuren, wurden von der Arbeitsgruppe um Krook beschrieben (Krook et al. (1998)).

Für die therapeutische Eignung als Pharmakon und/oder für den therapeutischen medizintechnischen Einsatz von Molekülen zur Bindung von Immunglobulinen der Klassen G₁₋₄, A und M sind die Spezifität für die genannten Immunglobulinklassen, eine geringe Bindung anderer Plasmabestandteile sowie die Stabilität des Peptids in Körperflüssigkeiten wie z.B. humanem Plasma oder Serum, elementare Voraussetzungen.

Der Erfindung liegt somit das technische Problem zugrunde, neue Peptide bereitzustellen, die eine hohe Affinität zu Immunglobulinen aufweisen.

Erfindungsgemäß wird dieses Problem durch synthetische Peptide gelöst, die vorzugsweise selektiv an Immunglobuline, insbesondere der Klassen G₁-₄, A und M in komplexen Lösungen, wie z. B. Körperflüssigkeiten und nativem humanen Blutplasma, binden und unter diesen Einsatzbedingungen stabil sind.

Die in diesem Patent beanspruchten Peptide erfüllen die genannten Anforderungen überraschenderweise sowohl in löslicher Form, als auch im immobilisiertem Zustand und sind somit für eine medizinisch-technische und pharmakologische Verwendung geeignet.

Insbesondere nach Immobilisierung der Peptide über X08 gleich K (Lys) (siehe unten) erfüllen die Peptide die genannten Forderungen, überraschenderweise auch nach thermischer Behandlung (Autoklavierung bei 121°C).

Im einzelnen sind dies die erfindungsgemäßen Peptide mit der folgenden Aminosäuresequenz sowie Peptide oder Proteine, die diese Aminosäuresequenz enthalten,

R¹-X01-X02-X03-X04-X05-X06-X07-X08-X09-X10-X11-X12-X13-R²,

wobei R¹, R² sowie X01 bis X013 die folgende Bedeutung haben:
- R¹ =: Amino-, Acetyl- sowie Spacer/Linker, oder Deletion
- X01 =: A, D, E, G, Deletion
- X02 =: C, S, D, E, Diaminobuttersäure (Dab), Diaminopropionsäure (Dpr), K, Ornithin (Orn),
- X03 =: A, S, T
- X04 =: E, F, H, K, M, R, S, T, V, W, Y
- X05 =: H
- X06 =: G, H, K, L, M, N, Q, R
- X07 =: D, G
- X08 =: D, H, K, M, N, Q, R
- X09 =: K, L, R
- X10 =: V
- X11 =: W
- X12 =: C, S, D, E, Diaminobuttersäure (Dab), Diaminopropionsäure (Dpr), K, Ornithin (Orn),
- X13 =: D, E, K, Q, R, S, T, Deletion
- R² =: -COOH, -Amid, -GK, -GKK, -(βA)GK, -(βA)GKK, -(βA)(βA), -(βA)(βA)K sowie Spacer/Linker, oder Deletion.

Die erfindungsgemäßen Peptide können insbesondere in linearer sowie zyklischer Form vorliegen, wobei der Peptidringschluss bei Anwesenheit von zwei Cysteinen über Disulfidbrückenbildung oder über eine Amidzyklisierung erfolgt, die gegebenenfalls über Seitenketten, über den C- und N-Terminus oder über eine Kombinationen beider Möglichkeiten erfolgt.

Erfindungsgemäß bevorzugt werden die folgenden Peptide,
R1-ACAWHLGKLVWCT-R2
R1-ECAWHLGKLVWCT-R2
R1-GCAWHLGKLVWCT-R2
R1--CAWHLGKLVWCT-R2
mit maximal vier gleichzeitigen Aminosäureaustauschen in den Peptidpositionen wie folgt,
- X02 =: S
- X03 =: S, T
- X04 =: E, F, H, K, M, R, S, T, V, Y
- X06 =: G, H, K, M, N, Q,R
- X07 =: D
- X08 =: D, H, M, N, Q, R
- X09 =: K, R
- X12 =: S
- X13 =: D, E, K, Q, R, S, Deletion
oder
R1-DSAWHLGKLVWCT-R2
mit maximal vier gleichzeitigen Aminosäureaustauschen in den Peptidpositionen wie folgt,
- X01 =: A, E, G, Deletion
- X03 =: S, T
- X04 =: E, F, H, K, M, R, S, T, V, Y
- X06 =: G, H, K, M, N, Q, R
- X07 =: D
- X08 =: D, H, M, N, Q, R
- X09 =: K, R
- X12 =: S
- X13 =: D, E, K, Q, R, S, Deletion
oder
R1-DCSWHLGKLVWCT-R2
R1-DCTWHLGKLVWCT-R2
mit maximal vier gleichzeitigen Aminosäureaustauschen in den Peptidpositionen wie folgt,
- X01 =: A, E, G, Deletion
- X02 =: S
- X04 =: E, F, H, K, M, R, S, T, V, Y
- X06 =: G, H, K, M, N, Q, R
- X07 =: D
- X08 =: D, H, M, N, Q, R
- X09 =: K, R
- X12 =: S
- X13 =: D, E, K, Q, R, S, Deletion,
oder
R1-DCAEHLGKLVWCT-R2
R1-DCAFHLGKLVWCT-R2
R1-DCAHHLGKLVWCT-R2
R1-DCAKHLGKLVWCT-R2
R1-DCAMHLGKLVWCT-R2
R1-DCARHLGKLVWCT-R2
R1-DCASHLGKLVWCT-R2
R1-DCATHLGKLVWCT-R2
R1-DCAVHLGKLVWCT-R2
R1-DCAWHLGKLVWCT-R2
R1-DCAYHLGKLVWCT-R2
mit maximal vier gleichzeitigen Aminosäureaustauschen in den Peptidpositionen wie folgt,
- X01 =: A, E, G, Deletion
- X02 =: S
- X03 =: S, T
- X06 =: G,H,K,M,N,Q,R
- X07 =: D
- X08 =: D, H, M, N, Q, R
- X09 =: K, R
- X12 =: S
- X13 =: D, E, K, Q, R, S, Deletion,
oder
R1-DCAWHDGKLVWCT-R2
R1-DCAWHEGKLVWCT-R2
R1-DCAWHGGKLVWCT-R2
R1-DCAWHHGKLVWCT-R2
R1-DCAWHKGKLVWCT-R2
R1-DCAWHMGKLVWCT-R2
R1-DCAWHNGKLVWCT-R2
R1-DCAWHQGKLVWCT-R2
R1-DCAWHRGKLVWCT-R2
mit maximal vier gleichzeitigen Aminosäureaustauschen in den Peptidpositionen wie folgt,
- X01 =: A, E, G, Deletion
- X02 =: S
- X03 =: S, T
- X04 =: E, F, H, K, M, R, S, T, V, Y
- X07 =: D
- X08 =: D, H, .M, N, Q, R
- X09 =: K, R
- X12 =: S
- X13 =: D, E, K, Q, R, S, Deletion,
oder
R1-DCAWHLDKLVWCT-R2
mit maximal vier gleichzeitigen Aminosäureaustauschen in den Peptidpositionen wie folgt,
- X01 =: A, E, G, Deletion
- X02 =: S
- X03 =: S, T
- X04 =: E, F, H, K, M, R, S, T, V, Y
- X06 =: G, H, K, M, N, Q, R
- X08 =: D, H, M, N, Q, R
- X09 =: K, R
- X12 =: S
- X13 =: D, E, K, Q, R, S, Deletion
oder
R1-DCAWHLGDLVWCT-R2
R1-DCAWHLGHLVWCT-R2
R1-DCAWHLGKLVWCT-R2
R1-DCAWHLGMLVWCT-R2
R1-DCAWHLGNLVWCT-R2
R1-DCAWHLGQLVWCT-R2
R1-DCAWHLGRLVWCT-R2
mit maximal vier gleichzeitigen Aminosäureaustauschen in den Peptidpositionen wie folgt,
- X01 =: A, E, G, Deletion
- X02 =: S
- X03 =: S, T
- X04 =: E, F, H, K, M, R, S, T, V, Y
- X06 =: G, H, K, M, N, Q, R
- X07 =: D
- X09 =: K, R
- X12 =: S
- X13 =: D, E, K, Q, R, S, Deletion,
oder
R1-DCAWHLGKKVWCT-R2
R1-DCAWHLGKRVWCT-R2
mit maximal vier gleichzeitigen Aminosäureaustauschen in den Peptidpositionen wie folgt,
- X01 =: A, E, G, Deletion
- X02 =: S
- X03 =: S, T
- X04 =: E, F, H, K, M, R, S, T, V, Y
- X06 =: G, H, K, M, N, Q, R
- X07 =: D
- X08 =: D, H, M, N, Q, R
- X12 =: S
- X13 =: D, E, K, Q, R, S, Deletion,
oder
R1-DCAWHLGKLVWST-R2
mit maximal vier gleichzeitigen Aminosäureaustauschen in den Peptidpositionen wie folgt,
- X01 =: A, E, G, Deletion
- X02 =: S
- X03 =: S, T
- X04 =: E, F, H, K, M, R, S, T, V, Y
- X06 =: G, H, K, M, N, Q, R
- X07 =: D
- X08 =: D, H, M, N, Q, R
- X09 =: K, R
- X13 =: D, E, K, Q, R, S, Deletion
oder
R1-DCAWHLGKLVWCD-R2
R1-DCAWHLGKLVWCE-R2
R1-DCAWHLGKLVWCK-R2
R1-DCAWHLGKLVWCQ-R2
R1-DCAWHLGKLVWCR-R2
R1-DCAWHLGKLVWCS-R2
R1-DCAWHLGKLVWC--R2
mit maximal vier gleichzeitigen Aminosäureaustauschen in den Peptidpositionen wie folgt,
- X01 =: A, E, G, Deletion
- X02 =: S
- X03 =: S, T
- X04 =: E, F, H, K, M, R, S, T, V, Y
- X06 =: G, H, K, M, N, Q, R
- X07 =: D
- X08 =: D, H, M, N, Q, R
- X09 =: K, R
- X12 =: S
wobei
R1 = Amino-, Acetyl- sowie Spacer/Linker,
R2 = -COOH, -Amid, -GK, -GKK, -(βA)GK, -(βA)GKK, -(βA)(βA), -(βA)(βA)K sowie Spacer/Linker sein kann, gegebenenfalls die Cysteine in Position X02 und X12 für einen Ringschluss über Disulfidbrückenbildung verwendet werden, oder die Cysteine in Position X02 und X12 durch Dpr oder Dab oder K oder Orn für X02 in Kombination mit D oder E für X12 ersetzt sind und für eine Amidzyklisierung über die Seitenketten verwendet werden,
oder die Cysteine in Position X02 und X12 durch D oder E für X02 in Kombination mit Dpr oder Dab oder K oder Orn für X12 ersetzt sind und für eine Amidzyklisierung über die Seitenketten verwendet werden,
oder bei R2 = COOH die Position X02 Dpr oder Dab, oder K oder Orn ist und deren Seitenkette mit der C-terminalen Aminosäure über eine Amidzyklisierung einen Ringschluss bildet,
oder bei R1 = NH2 die Position X12 E oder D ist und deren Seitenkette mit der N-terminalen Aminosäure über eine Amidzyklisierung einen Ringschluss bildet, oder bei R1 = NH2 und R2 = COOH die N-terminale und die C-terminale Aminosäure des Peptids für einen Ringschluss über Amidbildung verwendet wird,
und gegebenenfalls vorhandene Lysine, die nicht für einen Ringschluss verwendet werden, über Spacer/Linker derivatisiert sein können.

Des Weiteren werden Peptide erfindungsgemäß bevorzugt, die die folgenden Peptidsequenzen aufweisen und die linear oder gegebenenfalls über N-und C-Terminus des Peptids über eine Amidbindung zyklisiert sein können:
R1--CAWHLGKLVWCT-R2
R1-DCAWHLGKLVWC--R2
R1--CAWHLGKLVWC--R2
R1-DCSWHLGKLVWCT-R2
R1-DCAWHHGKLVWCT-R2
R1-DCAWHQGKLVWCT-R2
R1-DCAWHGGKLVWCT-R2
R1-DCAWHGGKLVWCT-R2
R1-DCAWHGGHLVWCT-R2
R1-DCAWHGGKLVWCE-R2
wobei R1 = Amino-, Acetyl-
R2 = -COOH, -Amid, -GK, -GKK, -(βA)GK, -(βA)GKK, -(βA)(βA), -(βA)(βA)K ist,
R1-DCAWHLGKLVWCT-R2
R1-DCAWHQGKLVWCT-R2
R1-DCAYHLGKLVWCT-R2
R1-DCSWHLGKLVWCT-R2
R1-DCAYHQGKLVWCT-R2
R1-DCSWHQGKLVWCT-R2
R1-DCSYHLGKLVWCT-R2
R1-DCSYHQGKLVWCT-R2
R1-CAWHLGKLVWCT-R2
R1--CAWHQGKLVWCT-R2
R1--CAYHLGKLVWCT-R2
R1-CSWHLGKLVWCT-R2
R1-CAYHQGKLVWCT-R2
R1--CSWHQGKLVWCT-R2
R1--CSYHLGKLVWCT-R2
R1--CSYHQGKLVWCT-R2
R1-DCAWHLGKLVWC--R2
R1-DCAWHQGKLVWC--R2
R1-DCAYHLGKLVWC--R2
R1-DCSWHLGKLVWC--R2
R1-DCAYHQGKLVWC--R2
R1-DCSWHQGKLVWC--R2
R1-DCSYHLGKLVWC--R2
R1-DCSYHQGKLVWC--R2
R1--CAWHLGKLVWC--R2
-R1--CAWHQGKLVWC--R2
R1--CAYHLGKLVWC--R2
R1--CSWHLGKLVWC--R2
R1--CAYHQGKLVWC--R2
R1--CSWHQGKLVWC--R2
R1--CSYHLGKLVWC--R2
R1--CSYHQGKLVWC--R2
wobei
R1 = Acetyl-
R2 = -COOH, -Amid ist,
gegebenenfalls die Cysteine in Position X02 und X12 für einen Ringschluss über Disulfdbrückenbildung verwendet werden,
oder die Cysteine in Position X02 und X12 durch Dpr oder Dab oder K oder Orn für X02 in Kombination mit D oder E für X12 ersetzt sind und für eine Amidzyklisierung über die Seitenketten verwendet werden,
oder die Cysteine in Position X02 und X12 durch D oder E für X02 in Kombination mit Dpr oder Dab oder K oder Orn für X12 ersetzt sind und für eine Amidzyklisierung über die Seitenketten verwendet werden.

Die genannten erfindungsgemäßen Peptide binden Immunglobuline oder Antigen-Immunglobulin-Komplexe in biologischen Flüssigkeiten.

Erfindungsgemäß beansprucht werden auch feste Phasen für die Affinitätschromatographie oder Festphasenextraktion aus organischen, anorganischen, synthetischen Polymeren oder aus Mischpolymeren, vorzugsweise quervernetzte Agarose, Cellulose, Kieselgel, Polyamid und Polyvinylalkohole, die gegebenenfalls chemisch aktiviert werden, mit an der Oberfläche der festen Phase immobilisierten erfindungsgemäßen Peptiden.

Bei den erfindungsgemäßen festen Phasen sind die Peptide vorzugsweise kovalent oder durch Adsorption an die feste Trägerphase gebunden. Bei einer weiteren bevorzugten Ausführungsform der erfindungsgemäßen festen Phasen sind die Peptide von der Trägeroberfläche über Linker/Spacer beabstandet. Um die erfindungsgemäßen Peptide an feste Phasen zu koppeln, werden diese vorzugsweise mit einem Linker/Spacer versehen. Vorzugsweise ist der Linker/Spacer ausgewählt aus der Gruppe bestehend aus:
Carbonsäuren und deren Derivate, Hydroxycarbonsäuren und deren Derivate, Oligoalkoxyderivate und deren Oligomere, α-Aminocarbonsäuren sowie deren Homo- und Heterooligomere, α,ω-Aminocarbonsäuren sowie deren verzweigte Homo- und Heterooligomere, sonstige Aminosäuren sowie deren lineare und verzweigte Homo- und Heterooligomere, Amino-oligoalkoxy-alkylamine und deren lineare und verzweigte Homo- und Heterooligomere, Maleinimidocarbonsäure und deren Derivate, Oligomere von Alkylaminen, 4-Alkylphenyl-Derivate, 4-Oligoalkoxyphenyl- und 4-Oligoalkoxyphenoxy-Derivate, Oligoalkylmercaptophenyl- und 4-Oligoalkylmercaptophenoxy-Derivate, 4-Oligoalkylaminphenyl- und 4-Oligoalkylaminyphenoxy-Derivate, (Oligoalkylbenzyl)-phenyl- bzw. 4-(Oligoalkylbenzyl)-phenoxy-Derivate sowie 4-(Oligoalkoxy-benzyl)-phenyl- bzw. 4-(Oligoalkoxybenzyl)-phenoxy-Derivate, Trityl-Derivate, Benzyloxyaryl- und Benzyloxyalkyl-Derivate, Xanthen-3-yl-oxyalkyl-Derivate, ω-(4-Alkylphenyl)- bzw. ω-(4-Alkylphenoxy)-alkansäure-Derivate, Oligoalkyl-phenoxyalkyl- bzw. Oligoalkoxy-phenoxyalkyl-Derivate, Carbamat-Derivate, Amine, Trialkylsilyl- und Dialkyl-alkoxysilyl-Derivate, Alkyl- bzw. Aryl-Derivate, Thiole und deren Derivate, Thioether und deren Derivate, Thiocarbonsäuren und deren Derivate, Thiolcarbonsäuren und deren Derivate, Sulfonsäuren und deren Derivate sowie Kombinationen aus den aufgeführten Linkern oder Spacern.

Gegenstand der Erfindung ist ein Verfahren zur Adsorption von Immunglobulinen an einer festen Phase, wobei die erfindungsgemäßen Peptide an eine für die Affinitätschromatographie übliche feste Phase oder an mobile feste Phasen gebunden werden, und die zu adsorbierenden immunglobulinhaltigen Proben insbesondere mit den erfindungsgemäßen festen Phasen in Kontakt gebracht werden.

Vorzugsweise wird ein Verfahren zur Adsorption von Immunglobulinen an einer festen Phase durchgeführt, wobei die erfindungsgemäßen Peptide an eine für die Affinitätschromatographie übliche feste Phase oder an mobile feste Phasen gebunden werden, und die zu adsorbierenden immunglobulinhaltigen Proben mit Antikoagulantien behandeltes humanes Blutplasma oder humanes Vollblut sind und mit den erfindungsgemäßen festen Phasen gebracht werden.

Das erfindungsgemäße Verfahren eignet sich insbesondere zur Adsorption von Immunglobulinen aus immunglobulinhaltigen Proben, die Autoantikörper enthalten, die mit Autoimmunerkrankungen, insbesondere rheumatioden Erkrankungen, Multiple Sklerose, Myasthenia gravis sowie andere Autoantikörper-vermittelte Erkrankungen, assoziiert sind.

Das erfindungsgemäße Verfahren wird vorteilhafterweise mit einer erfindungsgemäßen Vorrichtung zur Entfernung von Immunglobulinen aus immunglobulinhaltigen Proben an festen Phasen durchgeführt, wobei die Vorrichtung eine erfindungsgemäße feste Phase enthält und Einrichtungen zum Einlass von immunglobulinhaltigen Proben vorgesehen sind.

Gegenstand der Erfindung ist auch eine Verwendung der erfindungsgemäßen Peptide, der erfindungsgemäßen festen Phasen sowie der erfindungsgemäßen Vorrichtungen zur Entfernung von Immunglobulinen aus immunglobulinhaltigen Proben.
Die Erfindung wird an Hand der folgenden Beispiele näher erläutert.

### Beispiel 1

Peptidimmobilisierung auf Cellulosematrix und Test auf Immunglobulinbindung

### 1. Trägergebundene Synthese von 13-mer Peptidvarianten

Peptide bestehend aus 13 Aminosäuren wurden mittels Festphasensynthese (Houghten, 1985) an einer Cellulosematrix mit (βA)(βA) als Spacergruppierung synthetisiert.

Ausgehend von
R1-XO1-X02-X03-X04-X05-X06-X07-X08-X09-X10-X11-X12-X13-R2
- D - C - A - W - H - L - G - E - L - V - W - C - T -
R1 = Amino-
R2 = (βA)(βA), wobei βA = β-Alanin
wurden festphasenimmobilisierte Peptidvarianten synthetisiert wie in Tabelle 1 beschrieben.

### 2. Test der 13-mer Peptidvarianten auf Immunglobulin-Bindung

Die immobilisierten Peptide wurden auf ihre Bindungsfähigkeit von Immunglobulin durch Inkubation mit humanem IgG-Fc in gepufferter Lösung und durch Inkubation mit Humanplasma getestet.

Hierzu wurden die festphasenimmobilisierten Peptidvarianten mit T-TBS-Puffer (136 mM Nacl, 1,6 mM KCI, 0,05% (v/v) Tween 20, 50 mM Tris-HCl pH 8,0) gewaschen und anschließend mit Blockierungspuffer (5% (w/v) Saccharose, 4% (w/v) Rinderserumalbumin in T-TBS) behandelt. Die Inkubation der immobilisierten Peptide mit IgG-Fc (1 µg/ml) in Blockierungspuffer und Humanserum (1:10.000 verdünnt in Blockierungspuffer) erfolgte für 3 h bei Raumtemperatur. Zur Überprüfung der Spezifität der Bindung wurde gepufferte Serumalbumin-Lösung (Blockierungspuffer) ohne Zusatz als Kontrolle mitgeführt. Danach wurden die festphasenimmobilisierten Peptidvarianten noch dreimal mit T-TBS-Puffer gewaschen.

Nach Inkubation mit Alkalischer-Phosphatase-konjugiertem anti-Human IgG Antikörper (1,2 µg/ml) in Blockierungspuffer erfolgte die Detektion der Immunglobulinbindung anschließend mittels NBT (Nitroblautetrazoliumchlorid)/BCIP (5-Brom-4-Chlor-3-indolyl-phosphat, Toluidin-Salz) als Substrat (200 µl Stammlösung (18,75 mg/ml NBT und 9,4 mg/ml BCIP in 67% (v/v) DMSO) in 10 ml Entwicklungspuffer (100 mM Tris-HCl, 100 mM Nacl, 5 mM MgCl₂ pH 9,5)).

Die Ergebnisse der Tests auf Immunglotrulinbindung sind in Tabelle 1 dargestellt.

### Beispiel 2

Peptide immobilisiert auf Agarose-Beads mit Glu (E) im Vergleich zu Lys (K) in Peptidposition X08 (s.o.) und Test der Immunglobulin-Bindung aus Humanplasma

### 1. Immobilisierung

Zur Immobilisierung an einer festen Phase wurden die Peptide zu einer Konzentration von 1 mg/ml in Kopplungspuffer (30 % (v/v) Acetonitril, 0,5 M NaCl, 0,1 M NaHCO₃ pH 8,3) gelöst und mit gewaschener und CNBrvoraktivierter Sepharose 4B im Volumenverhältnis 10 : 1 gemischt. Nach Beendigung der Kopplungsreaktion wurden die Peptidmatrizes mit Kopplungspuffer gewaschen und die überschüssigen CNBr-Gruppen durch Inkubation der Matrix in Tris-Puffer (100 mM Tris-HCl, 500 mm NaCl pH 8,0) inaktiviert.

Die Peptidbeladung der Matrizes wurde photometrisch (A₂₈₀ₙₘ) durch Differenzbildung der eingesetzten Peptidmasse vor und der nichtimmobilisierten Peptidmasse nach der Kopplung bestimmt.

Die Ergebnisse der Peptidbeladungen sind in Tabelle 2 dargestellt.

### Immobilisierte Peptide:

| | |
|---|---|
| A | Lineare Peptide |
| A1 | Acetyl- D-S-A-W-H-L-G-E-L-V-W-C-T -((βA)(βA)K) |
| B | Peptide zyklisiert über Disulfidbrückenbindung |
| B1 | 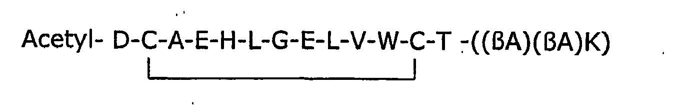 |
| B2 | 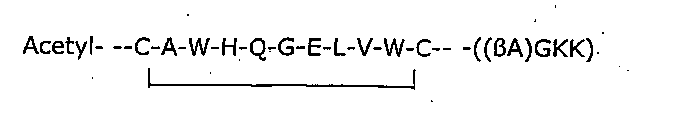 |
| B3 | 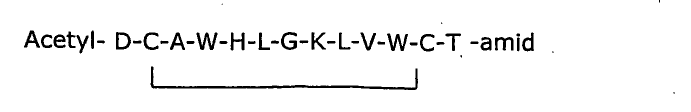 |
| C | Peptide zyklisiert über Seitenkettenamidbindung |
| C1 | 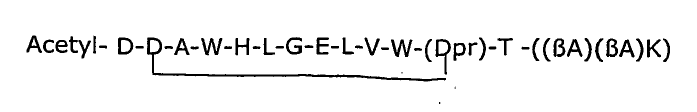 |
| C2 | 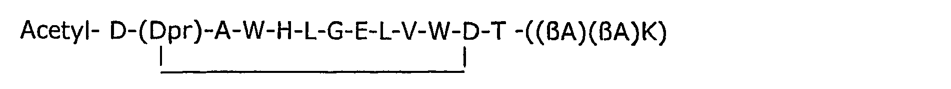 |

| | |
|---|---|
| Dpr = Diaminopropionsäure | |

### 2. Immunglobulinbindung aus Humanplasma

Die Peptidmatrizes wurden mittels Affinitätschromatographie auf ihre Bindungskapazität und auf ihre Bindungsspezifität für Immunglobuline mit Humanplasma als Probe getestet. Hierzu wurden als Probe 6 Volumenanteile Humanplasma mit einer linearen Flussrate von 80 cm/h über 1 Säulenvolumenanteil Peptidmatrix geleitet.

Vor Probenauftrag wurde die Affinitätschromatographiesäule mit PBS pH 7,2 äquilibriert worden. Nach Probenauftrag wurde die Säule mit PBS pH 7,2 bis zur Erreichung der Adsorptionsbasislinie gespült und anschließend das gebundene Protein mit 30 mM Na-Citratpuffer pH 2,8 eluiert.

Die Konzentrationsbestimmung des eluierten Proteins erfolgte durch photometrische Messung der Adsorption bei 280 nm. Die Immunglobulinkonzentration wurde daraus mit ε 280 nm = 1,35 cm²/mg für IgG berechnet.

Die Ergebnisse der Kapazitätsbestimmungen sind in Tabelle 2 dargestellt.

Die Spezifität der Immunglobulin-Peptidbindung wurde mittels SDS-PAGE ermittelt. Sie wurde mit der Spezifität für Immunglobulin von Protein A und anti-Immunglobulin G-Antikörpern verglichen.

Hierzu wurden die Eluate des jeweiligen Affinitätschromatographielaufs (wie oben beschrieben) unter reduzierenden Bedingungen für die SDS-PAGE aufbereitet.

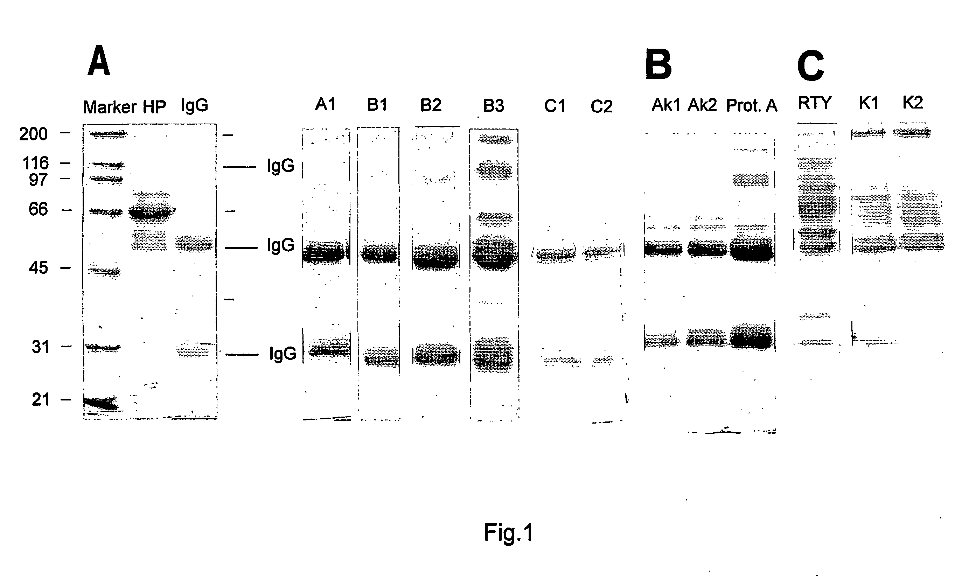

Die Ergebnisse der Spezifitätsprüfung sind in Fig. 1 dargestellt.

### Beispiel 3

Glu (E) im Vergleich zu Lys (K) in Peptidposition X08 (s.o.): Peptidimmobilisierung auf Agarose-Beads und Test der Immunglobulin-Bindung aus Humanplasma vor und nach thermischer Behandlung

### 1. Immobilisierung

Zur Immobilisierung an einer festen Phase wurden die Peptide zu einer Konzentration von 1 mg/ml in Kopplungspuffer (30 % (v/v) Acetonitril, 0,5 M Nacl, 0,1 M NaHC0₃ pH 8,3) gelöst und mit gewaschener und CNBrvoraktivierter Sepharose 4B im Volumenverhältnis 10 : 1 gemischt. Nach Beendigung der Kopplungsreaktion wurden die Peptidmatrizes mit Kopplungspuffer gewaschen und die überschüssigen CNBr-Gruppen durch Inkubation der Matrix in Tris-Puffer (100 mM Tris-HCl, 500 mM NaCl pH 8,0) inaktiviert.

Die Peptidbeladung der Matrizes wurde photometrisch (A280nm) durch Differenzbildung der eingesetzten Peptidmasse vor und der nichtimmobilisierten Peptidmasse nach der Kopplung bestimmt.

### Immobilisierte Peptide:

Alle genannten Peptide sind über Disulfidbrückenbindung zyklisiert.

| | |
|---|---|
| MF0147 | 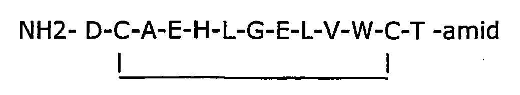 |

Immobilisierung über den Aminoterminus.

| | |
|---|---|
| MF0146 | 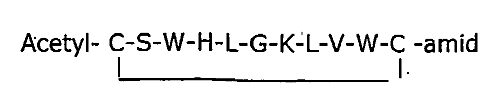 |

Immobilisierung über die e-Aminogruppe des Lysins (K)

| | |
|---|---|
| MF0143 | 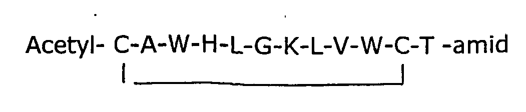 |

Immobilisierung über die e-Aminogruppe des Lysins (K)

### 2. Immunglobulinbindung aus Humanplasma vor und nach Autoklavierung der Peptidmatrices

Die Peptidmatrizes wurden vor und nach Autoklavierung bei 121°C für 20 min mittels Affinitätschromatographie auf ihre Bindungskapazität und auf ihre Bindungsspezifität für Immunglobuline mit Humanplasma als Probe getestet. Hierzu wurden als Probe 6 Volumenanteile Humanplasma mit einer linearen Flussrate von 80 cm/h über 1 Säulenvolumenanteil Peptidmatrix geleitet.

Vor Probenauftrag wurde die Affinitätschromatographiesäule mit. PBS pH 7,2 equilibriert worden. Nach Probenauftrag wurde die Säule mit PBS pH 7,2 bis zur Erreichung der Adsorptionsbasislinie gespült und anschließend das gebundene Protein mit 30 mM Na-Citratpuffer pH 2,8 eluiert.

Die Konzentrationsbestimmung des eluierten Proteins erfolgte durch photometrische Messung der Adsorption bei 280 nm. Die Immunglobulinkonzentration wurde daraus mit ε 280 nm = 1,35 cm²/mg für IgG berechnet.

Die Ergebnisse der Kapazitätsbestimmungen sind in Tabelle 3 dargestellt.

Die Spezifität der Immunglobulin-Peptidbindung vor und nach Autoklavierung wurde mittels SDS-PAGE ermittelt.

Hierzu wurden die Eluate des jeweiligen Affinitätschromatographielaufs (wie oben beschrieben) unter reduzierenden Bedingungen für die SDS-PAGE aufbereitet.

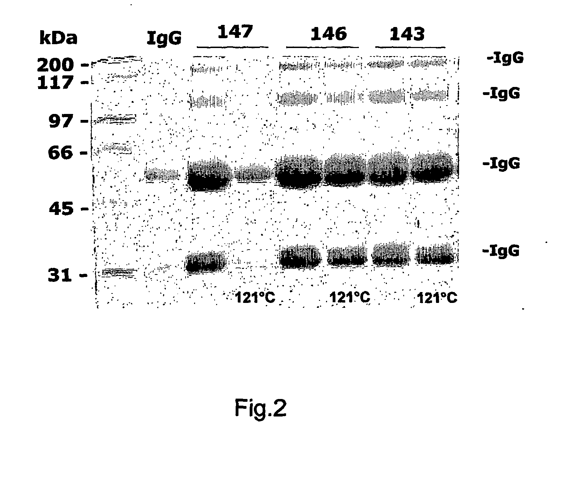

Die Ergebnisse der Spezifitätsprüfung sind in Fig. 2 dargestellt.

### Tabelle 1: Test von 248 Peptidvarianten (13-mere) auf Immunglobulin-Bindung

(A) Inkubation der festphasenimmobilisierten Peptidvarianten mit Serumalbumin als negative Kontrollprobe
(B) Inkubation der festphasenimmobilisierten Peptidvarianten mit Humanplasma als Probe
(C) Inkubation der festphasenimmobilisierten Peptidvarianten mit humanem IgG-Fc als Probe

Die Detektion der Bindung von Immunglobulinen an die festphasenimmobilisierten Peptidvarianten erfolgte mittels Alkalischer-Phosphatase-konjugiertem anti-Human IgG Antikörper.

Erste Zeile: Ein-Buchstaben-Code für die ausgetauschte Aminosäure

Erste Spalte: Sequenzposition der ausgetauschten Aminosäure

Keine Bindung von Immunglobulin bei vorgegebener Peptidvariante, Detektion negativ: 0

Bindung von Immunglobulin bei vorgegebener Peptidvariante, Detektion positiv: +

### (A) Ergebnis bei Inkubation mit Serumalbumin:

| | A | C | D | E | F | G | H | I | J | K | L | M | N | P | Q | R | S | T | V | W | Y |
|---|---|---|---|---|---|---|---|---|---|---|---|---|---|---|---|---|---|---|---|---|---|
| 01 | 0 | 0 | 0 | 0 | 0 | 0 | 0 | 0 | 0 | 0 | 0 | 0 | 0 | 0 | 0 | 0 | 0 | 0 | 0 | 0 | 0 |
| 02 | 0 | 0 | 0 | 0 | 0 | 0 | 0 | 0 | 0 | 0 | 0 | 0 | 0 | 0 | 0 | 0 | 0 | 0 | 0 | 0 | 0 |
| 03 | 0 | 0 | 0 | 0 | 0 | 0 | 0 | 0 | 0 | 0 | 0 | 0 | 0 | 0 | 0 | 0 | 0 | 0 | 0 | 0 | 0 |
| 04 | 0 | 0 | 0 | 0 | 0 | 0 | 0 | 0 | 0 | 0 | 0 | 0 | 0 | 0 | 0 | 0 | 0 | 0 | 0 | 0 | 0 |
| 05 | 0 | 0 | 0 | 0 | 0 | 0 | 0 | 0 | 0 | 0 | 0 | 0 | 0 | 0 | 0 | 0 | 0 | 0 | 0 | 0 | 0 |
| 06 | 0 | 0 | 0 | 0 | 0 | 0 | 0 | 0 | 0 | 0 | 0 | 0 | 0 | 0 | 0 | 0 | 0 | 0 | 0 | 0 | 0 |
| 07 | 0 | 0 | 0 | 0 | 0 | 0 | 0 | 0 | 0 | 0 | 0 | 0 | 0 | 0 | 0 | 0 | 0 | 0 | 0 | 0 | 0 |
| 08 | 0 | 0 | 0 | 0 | 0 | 0 | 0 | 0 | 0 | 0 | 0 | 0 | 0 | 0 | 0 | 0 | 0 | 0 | 0 | 0 | 0 |
| 09 | 0 | 0 | 0 | 0 | 0 | 0 | 0 | 0 | 0 | 0 | 0 | 0 | 0 | 0 | 0 | 0 | 0 | 0 | 0 | 0 | 0 |
| 10 | 0 | 0 | 0 | 0 | 0 | 0 | 0 | 0 | 0 | 0 | 0 | 0 | 0 | 0 | 0 | 0 | 0 | 0 | 0 | 0 | 0 |
| 11 | 0 | 0 | 0 | 0 | 0 | 0 | 0 | 0 | 0 | 0 | 0 | 0 | 0 | 0 | 0 | 0 | 0 | 0 | 0 | 0 | 0 |
| 12 | 0 | 0 | 0 | 0 | 0 | 0 | 0 | 0 | 0 | 0 | 0 | 0 | 0 | 0 | 0 | 0 | 0 | 0 | 0 | 0 | 0 |
| 13 | 0 | 0 | 0 | 0 | 0 | 0 | 0 | 0 | 0 | 0 | 0 | 0 | 0 | 0 | 0 | 0 | 0 | 0 | 0 | 0 | 0 |

### (B) Ergebnis bei Inkubation mit Humanplasma:

| | A | C | D | E | F | G | H | I | K | L | M | N | P | Q | R | S | T | V | W | Y |
|---|---|---|---|---|---|---|---|---|---|---|---|---|---|---|---|---|---|---|---|---|
| 01 | + | 0 | + | + | 0 | + | 0 | 0 | 0 | 0 | 0 | 0 | 0 | 0 | 0 | 0 | 0 | 0 | 0 | 0 |
| 02 | 0 | + | 0 | 0 | 0 | 0 | 0 | 0 | 0 | 0 | 0 | 0 | 0 | 0 | 0 | 0 | 0 | 0 | 0 | 0 |
| 03 | + | 0 | 0 | 0 | 0 | 0 | 0 | 0 | 0 | 0 | 0 | 0 | 0 | 0 | 0 | + | + | 0 | 0 | 0 |
| 04 | 0 | 0 | 0 | + | + | 0 | + | 0 | + | 0 | + | 0 | 0 | 0 | + | + | + | + | + | + |
| 05 | 0 | 0 | 0 | 0 | 0 | 0 | + | 0 | 0 | 0 | 0 | 0 | 0 | 0 | 0 | 0 | 0 | 0 | 0 | 0 |
| 06 | 0 | 0 | 0 | 0 | 0 | + | + | 0 | + | + | + | + | 0 | + | + | 0 | 0 | 0 | 0 | 0 |
| 07 | 0 | 0 | + | 0 | 0 | + | 0 | 0 | 0 | 0 | 0 | 0 | 0 | 0 | 0 | 0 | 0 | 0 | 0 | 0 |
| 08 | 0 | 0 | + | + | 0 | 0 | + | 0 | + | 0 | + | + | 0 | + | + | 0 | 0 | 0 | 0 | 0 |
| 09 | 0 | 0 | 0 | 0 | 0 | 0 | 0 | 0 | + | + | 0 | 0 | 0 | 0 | + | 0 | 0 | 0 | 0 | 0 |
| 10 | 0 | 0 | 0 | 0 | 0 | 0 | 0 | 0 | 0 | 0. | 0 | 0 | 0 | 0 | 0 | 0 | 0 | + | 0 | 0 |
| 11 | 0 | 0 | 0 | 0 | 0 | 0 | 0 | 0 | 0 | 0 | 0 | 0 | 0 | 0 | 0 | 0 | 0 | 0 | + | 0 |
| 12 | 0 | + | 0 | 0 | 0 | 0 | 0 | 0 | 0 | 0 | 0 | 0 | 0 | 0 | 0 | 0 | 0 | 0 | 0 | 0 |
| 13 | 0 | 0 | + | + | 0 | 0 | 0 | 0 | + | 0 | 0 | 0 | 0 | + | + | + | + | 0 | 0 | 0 |

### (C) Ergebnis bei Inkubation mit human IgG-Fc:

| | A | C | D | E | F | G | H | I | K | L | M | N | P | Q | R | S | T | V | W | Y |
|---|---|---|---|---|---|---|---|---|---|---|---|---|---|---|---|---|---|---|---|---|
| 01 | + | 0 | + | + | 0 | + | + | 0 | + | + | + | + | + | + | + | + | 0 | 0 | 0 | 0 |
| 02 | 0 | + | 0 | 0 | 0 | 0 | 0 | 0 | 0 | 0 | 0 | 0 | 0 | 0 | 0 | 0 | 0 | 0 | 0 | 0 |
| 03 | + | 0 | 0 | + | 0 | 0 | 0 | 0 | + | 0 | 0 | 0 | 0 | + | 0 | + | + | 0 | 0 | 0 |
| 04 | 0 | 0 | 0 | + | + | 0 | 0 | + | + | + | + | + | + | 0 | + | + | + | + | + | + |
| 05 | 0 | 0 | 0 | 0 | 0 | 0 | + | 0 | 0 | 0 | 0 | 0 | 0 | 0 | 0 | 0 | 0 | 0 | 0 | 0 |
| 06 | + | 0 | + | + | 0 | + | + | 0 | + | + | + | + | 0 | + | + | + | + | 0 | 0 | 0 |
| 07 | 0 | 0 | + | 0 | 0 | + | 0 | 0 | 0 | 0 | 0 | 0 | 0 | 0 | 0 | 0 | 0 | 0 | 0 | 0 |
| 08 | + | 0 | + | + | 0 | 0 | + | 0 | + | 0 | + | + | 0 | + | + | + | 0 | 0 | 0 | 0 |
| 09 | 0 | 0 | 0 | 0 | 0 | 0 | 0 | 0 | + | + | + | 0 | + | + | + | 0 | 0 | 0 | 0 | 0 |
| 10 | 0 | 0 | 0 | 0 | 0 | 0 | 0 | 0 | 0 | 0 | 0 | 0 | 0 | 0 | 0 | 0 | 0 | + | 0 | 0 |
| 11 | 0 | 0 | 0 | 0 | 0 | 0 | + | 0 | + | 0 | 0 | 0 | 0 | 0 | 0 | 0 | | 0 | 0 | + 0 |
| 12 | 0 | + | 0 | 0 | 0 | 0 | 0 | 0 | 0 | 0 | 0 | 0 | 0 | 0 | 0 | 0 | 0 | 0 | 0 | 0 |
| 13 | + | 0 | + | + | 0 | 0 | + | 0 | + | 0 | + | + | 0 | + | + | + | + | 0 | 0 | 0 |

### Tabelle 2: Bindungskapazität der Peptidmatrizes für Immunglobuline

Beladung: mg immobilisiertes Peptid/ ml Matrix [mg/ml].
Volumenkapazität: mg gebundenes Protein / ml Peptidmatrix; [mg/ml].
Relative Kapazität: mg gebundenes Protein / mg Peptidmatrixbeladung; [mg/mg].

| Peptidmatrix | Beladung | Volumenkapazität | Rel. Kapazität |
|---|---|---|---|
| A1 | 8,8 | 4,70 | 0,53 |
| B1 | 8,2 | 1,18 | 0,14 |
| B2 | 8,5 | 5,87 | 0,69 |
| B3 | 7,7 | 10,7 | 1,40 |
| C1 | 8,0 | 1,74 | 0,22 |
| C2 | 8,8 | 1,50 | 0,17 |

### Fig. 1: Spezifität der Immunglobulin-Peptidbindung

Die Spezifität der Immunglobulin-Peptidbindung (A) wurde im SDS-PAGE mit der Spezifität der Immunglobulin-Protein A-Bindung (Prot. A) und mit der Spezifität der Bindung von Human-IgG mit einem Huhn-IgY-anti Human-IgG Antikörper (AK1) bzw. einem Schaf-IgG-anti Human-IgG Antikörper (AK2) verglichen (B) sowie mit der Spezifität der Immunglobulin-Peptid RTY-Bindung, (Acetyl-(RTy)βA))₂KGKK-amid abgeleitet von Fassina et al.(1996) und der Spezifität der Immunglobulin-Peptid K1- bzw. K2-Bindung, wobei K1 Acetyl-FGRLVSSIRY(βA)(βA)K-amid ist und K2 Acetyl-TWKTSRISIF(βA)(βA)K-amid ist, abgeleitet von Krook et al. (1998) (C).

### (A)

- Marker:: Marker für die molekularen Masse, in kDa.
- HP:: Humanplasmaprobe, 10 µl Probe 1:200 in PBS vorverdünnt.
- IgG:: Humanes Immunglobulin G, 1,25 µg.

### Lineare Peptide

- A1:: Eluat von der Peptidmatrix A1, 10 µl Probe

### Peptide zyklisiert über Disulfidbrückenbindung

- B1:: Eluat von der Peptidmatrix B1, 10 µl Probe
- B2:: Eluat von der Peptidmatrix B2, 10 µl Probe
- B3:: Eluat von der Peptidmatrix B3, 10 µl Probe

### Peptide zyklisiert über Seitenkettenamidbindung

- C1:: Eluat von der Peptidmatrix C1, 10 µl Probe
- C2:: Eluat von der Peptidmatrix C2, 10 µl Probe

### (B)

- Ak1:: Eluat von der Antikörpermatrix 1, 10 µl Probe
- Ak2:: Eluat von der Antikörpermatrix 2, 10 µl Probe
- Prot. A:: Eluat von der Protein A-Matrix, 10 µl Probe

### (C)

- RTY:: Eluat von der Peptidmatrix RTY, 10 µl Probe
- K1:: Eluat von der Peptidmatrix K1, 10 µl Probe
- K2:: Eluat von der Peptidmatrix K2, 10 µl Probe

Die SDS-PAGE Probenvorbereitung erfolgte unter reduzierten Bedingungen.

**Tabelle 3: Bindungskapazität der Peptidmatrizes für Immunglobuline vor und nach Autoklavierung**

| Peptidmatrix | Rel. Kapazität vor Autoklavierung | [% ] | Rel. Kapazität nach Autoklavierung | [% ] | Relative Restkapazität bezogen auf MF147 |
|---|---|---|---|---|---|
| MF0147 | 5,11 | 100 | 0,72 | 14 | 100% |
| MF0146 | 3,48 | 100 | 1,88 | 54 | 386% |
| MF0143 | 2,85 | 100 | 1,85 | 65 | 464% |

Relative Kapazität: mg gebundenes Protein / mg Peptidmatrixbeladung; [mg/mg].

Fig. 2: Spezifität der Immunglobulin-Peptidbindung vor und nach Autoklavierung
- Marker:: Marker für die molekularen Masse, in kDa.
- IgG:: Humanes Immunglobulin G, 1,25 µg.
- 147, 146, 143:: Eluat der jeweiligen Peptidmatrix vor Autoklavierung, jeweils 10 µl Probe
- 121°C:: Eluat nach Autoklavierung der Matrix bei 121°C für 20 min, jeweils 10 µl Probe

SDS-PAGE Probenvorbereitung erfolgte unter reduzierenden Bedingungen.

### Literatur

Davies DR, Metzger H. Structural basis of antibody function. Annu Rev Immunol 1983;1:87-117
Alt FW, Blackwell TK, Yancopoulos GD Development of the primary antibody repertoire. Science 1987 Nov 20;238(4830):1079-87
McKenzie SE, Schreiber AD. Biological advances and clinical applications of Fc receptors for IgG. Curr Opin Hematol 1994 Jan;1(1):45-52
Makiya R, Stigbrand T., Placental alkaline phosphatase as the placental IgG receptor. Clin Chem 1992 Dec;38(12):2543-5
Sarfati M, Fournier S, Wu CY, Delespesse G., Expression, regulation and function of human Fc epsilon RII (CD23) antigen. Immunol Res. 1992;11(3-4):260-72
Capron M, Truong MJ, Aldebert D, Gruart V, Suemura M, Delespesse G, Tourvieille B, Capron A., Eosinophil IgE receptor and CD23 Immunol Res. 1992;11(3-4):252-9
Shen L, Receptors for IgA on phagocytic cells.Immunol Res. 1992;11(3-4):273-82. Review.
Sandor M, Ibraghimov A, Rosenberg MG, Teeraratkul P, Lynch RG, Expression of IgA and IgM Fc receptors on murine T lymphocytes. Immunol Res. 1992;11(3-4):169-80.
Miletic VD, Frank MM, Complement-immunoglobulin interactions. Curr Opin Immunol. 1995 Feb;7(1):41-7
Stahl S, Nygren PA, Sjolander A, Uhlen M, Engineered bacterial receptors in immunology. Curr Opin Immunol. 1993 Apr;5(2):272-7
Belak M, Borberg H, Jimenez C, Oette K, Technical and clinical experience with protein A immunoadsorption columns. Transfus Sci 1994 Dec; 15(4):419-22
Samuelsson G, Immunoadsorption using the Excorim treatment system. Transfus Sci 1998 Mar;19 Suppl:3-4
Felson DT, LaValley MP, Baldassare AR, Block JA, Caldwell JR, Cannon GW, Deal C, Evans S, Fleischmann R, Gendreau RM, Harris ER, Matteson EL, Roth SH, Schumacher HR, Weisman MH, Furst DE, The Prosorba column for treatment of refractory rheumatoid arthritis: a randomized, double-blind, sham-controlled trial. Arthritis Rheum 1999 Oct;42(10):2153-9
Koll RA, Ig-Therasorb immunoadsorption for selective removal of human immunoglobulins in diseases associated with pathogenic antibodies of all classes and IgG subclasses, immune complexes, and fragments of immunoglobulins. Ther Apher 1998 May;2(2):147-52
Knobl P, Derfler K, Extracorporeal immunoadsorption for the treatment of haemophilic patients with inhibitors to factor VIII or IX. Vox Sang 1999;77 Suppl 1:57-64
Jimenez C, Rosenow F, Grieb P, Haupt WF, Borberg H, Adsorption therapy with tryptophan-conjugated polyvinyl alcohol gels in 10 patients with acute Guillain-Barre syndrome. Transfus Sci 1993 Jan; 14(1):9-11
Fadul JE, Danielson BG, Wikstrom B, Reduction of plasma fibrinogen, immunoglobulin G, and immunoglobulin M concentrations by immunoadsorption therapy with tryptophan and phenylalanine adsorbents. Artif Organs 1996 Sep;20(9):986-90
Takamori M, Ide Y, Specific removal of anti-acetylcholine receptor antibodies in patients with myasthenia gravis. Transfus Sci 1996 Sep;17(3):445-53
Fassina G, Verdoliva A, Odierna MR, Ruvo M, Cassini G, Protein A mimetic peptide ligand for affinity purification of antibodies. J Mol Recognit 1996 Sep-Dec;9(5-6):564-9
Fassina G, Verdoliva A, Palombo G, Ruvo M, Cassani G, Immunoglobulin specificity of TG19318: a novel synthetic ligand for antibody affinity purification. J Mol Recognit 1998 Winter;11(1-6): 128-33
DeLano WL, Ultsch MH, de Vos AM, Wells JA, Convergent solutions to binding at a protein-protein interface. Science 2000 Feb 18;287(5456):1279-83
Krook M, Mosbach K, Ramstrom O, Novel peptides binding to the Fc-portion of immunoglobulins obtained from a combinatorial phage display peptide library. J Immunol Methods 1998 Dec 1;221(1-2):151-7
Houghten RA, General method for the rapid solid-phase synthesis of large numbers of peptides: specificity of antigen-antibody interaction at the level of individual amino acids. Proc Natl Acad Sci U S A 1985 Aug;82(15):5131-5.

## Patentansprüche

1. Peptide mit der folgenden Aminosäuresequenz,
R¹-X01-X02-X03-X04-X05-X06-X07-X08-X09-X10-X11-X12-X13-R²,
wobei R¹, R² sowie X01 bis X013 die folgende Bedeutung haben:
R¹ = Amino-, Acetyl- sowie Spacer/Linker, oder Deletion
X01 = A, D, E, G, Deletion
X02 = C, S, D, E, Diaminobuttersäure (Dab), Diaminopropionsäure (Dpr), K, Ornithin (Orn),
X03 = A, S, T
X04 = E, F, H, K, M, R, S, T, V, W, Y
X05 = H
X06 = E, G, H, K, L, M, N, Q, R
X07 = D, G
X08 = D, H, K, M, N, Q, R
X09 = K, L, R
X10 = V
X11 = W
X12 = C, S, D, E, Diaminobuttersäure (Dab), Diaminopropionsäure (Dpr), K, Ornithin (Orn),
X13 = D, E, K, Q, R, S, T, Deletion
R2 = -COOH, -Amid, -GK, -GKK, -(βA)GK, -(βA)GKK, -(βA)(βA), -(βA)(βA)K sowie Spacer/Linker, oder Deletion.

2. Peptid nach Anspruch 1 in linearer sowie zyklischer Form, wobei der Peptidringschluss bei Anwesenheit von zwei Cysteinen über Disulfidbrückenbildung oder über eine Amidzyklisierung erfolgt, die gegebenenfalls über Seitenketten, über den C- und N-Terminus oder über eine Kombinationen beider Möglichkeiten erfolgt.

3. Peptide nach Anspruch 1 und/oder 2, **dadurch gekennzeichnet, dass** es sich um
a) die folgenden Peptidsequenzen handelt,
R1-ACAWHLGKLVWCT-R2
R1-ECAWHLGKLVWCT-R2
R1-GCAWHLGKLVWCT-R2
R1--CAWHLGKLVWCT-R2
mit maximal vier gleichzeitigen Aminosäureaustauschen in den Peptidpositionen wie folgt,
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, Deletion
oder
R1-DSAWHLGKLVWCT-R2
mit maximal vier gleichzeitigen Aminosäureaustauschen in den Peptidpositionen wie folgt,
X01 = A, E, G, Deletion
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, Deletion
oder
R1-DCSWHLGKLVWCT-R2
R1-DCTWHLGKLVWCT-R2
mit maximal vier gleichzeitigen Aminosäureaustauschen in den Peptidpositionen wie folgt,
X01 = A, E, G, Deletion
X02 = S
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, Deletion,
oder
R1-DCAEHLGKLVWCT-R2
R1-DCAFHLGKLVWCT-R2
R1-DCAHHLGKLVWCT-R2
R1-DCAKHLGKLVWCT-R2
R1-DCAMHLGKLVWCT-R2
R1-DCARHLGKLVWCT-R2
R1-DCASHLGKLVWCT-R2
R1-DCATHLGKLVWCT-R2
R1-DCAVHLGKLVWCT-R2
R1-DCAWHLGKLVWCT-R2
R1-DCAYHLGKLVWCT-R2
mit maximal vier gleichzeitigen Aminosäureaustauschen in den Peptidpositionen wie folgt,
X01 = A, E, G, Deletion
X02 = S
X03 = S, T
X06 = G, H, K, M, N, Q, R
X07 = D
X08 = D, H, M, N, Q, R
X09 = K, R
X12 = S
X13 = D, E, K, Q, R, S, Deletion,
oder
R1-DCAWHDGKLVWCT-R2
R1-DCAWHEGKLVWCT-R2
R1-DCAWHGGKLVWCT-R2
R1-DCAWHHGKLVWCT-R2
R1-DCAWHKGKLVWCT-R2
R1-DCAWHMGKLVWCT-R2
R1-DCAWHNGKLVWCT-R2
R1-DCAWHQGKLVWCT-R2
R1-DCAWHRGKLVWCT-R2
mit maximal vier gleichzeitigen Aminosäureaustauschen in den Peptidpositionen wie folgt,
X01 = A, E, G, Deletion
X02 = S
X03 = S, T
X04 = E, F, H, K, M, R, S, T, V, Y
X07 = D
X08 = D, H, M, N, Q, R
X09 = K, R
X12 = S
X13 = D, E, K, Q, R, S, Deletion,
oder
R1-DCAWHLDKLVWCT-R2
mit maximal vier gleichzeitigen Aminosäureaustauschen in den Peptidpositionen wie folgt,
X01 = A, E, G, Deletion
X02 = S
X03 = S, T
X04 = E, F, H, K, M, R,S,T,V,Y
X06 = G, H, K, M, N, Q, R
X08 = D, H, M, N, Q, R
X09 = K, R
X12 = S
X13 = D, E, K, Q, R, S, Deletion
oder
R1-DCAWHLGDLVWCT-R2
R1-DCAWHLGHLVWCT-R2
R1-DCAWHLGKLVWCT-R2
R1-DCAWHLGMLVWCT-R2
R1-DCAWHLGNLVWCT-R2
R1-DCAWHLGQLVWCT-R2
R1-DCAWHLGRLVWCT-R2
mit maximal vier gleichzeitigen Aminosäureaustauschen in den Peptidpositionen wie folgt,
X01 = A, E, G, Deletion
X02 = S
X03 = S, T
X04 = E, F, H, K, M, R, S, T, V, Y
X06 = G, H, K, M, N, Q, R
X07 = D
X09 = K, R
X12 = S
X13 = D, E, K, Q, R, S, Deletion,
oder
R1-DCAWHLGKKVWCT-R2
R1-DCAWHLGKRVWCT-R2
mit maximal vier gleichzeitigen Aminosäureaustauschen in den Peptidpositionen wie folgt,
X01 = A, E, G, Deletion
X02 = S
X03 = S, T
X04 = E, F, H, K, M, R, S, T, V, Y
X06 = G, H, K, M, N, Q, R
X07 = D
X08 = D, H, M, N, Q, R
X12 = S
X13 = D, E, K, Q, R, S, Deletion,
oder
R1-DCAWHLGKLVWST-R2
mit maximal vier gleichzeitigen Aminosäureaustauschen in den Peptidpositionen wie folgt,
X01 = A, E, G, Deletion
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X13 = D, E, K, Q, R, S, Deletion
oder
R1-DCAWHLGKLVWCD-R2
R1-DCAWHLGKLVWCE-R2
R1-DCAWHLGKLVWCK-R2
R1-DCAWHLGKLVWCQ-R2
R1-DCAWHLGKLVWCR-R2
R1-DCAWHLGKLVWCS-R2
R1-DCAWHLGKLVWC--R2
mit maximal vier gleichzeitigen Aminosäureaustauschen in den Peptidpositionen wie folgt,
X01 = A, E, G, Deletion
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
wobei
R1 = Amino-, Acetyl- sowie Spacer/Linker,
R2 = -COOH, -Amid, -GK, -GKK, -(βA)GK, -(βA)GKK, -(βA)(βA), -(βA)(βA)K sowie Spacer/Linker sein kann, gegebenenfalls die Cysteine in Position X02 und X12 für einen Ringschluss über Disulfidbrückenbildung verwendet werden, oder die Cysteine in Position X02 und X12 durch Dpr oder Dab oder K oder Orn für X02 in Kombination mit D oder E für X12 ersetzt sind und für eine Amidzyklisierung über die Seitenketten verwendet werden,
oder die Cysteine in Position X02 und X12 durch D oder E für X02 in Kombination mit Dpr oder Dab oder K oder Orn für X12 ersetzt sind und für eine Amidzyklisierung über die Seitenketten verwendet werden,
oder bei R2 = COOH die Position X02 Dpr oder Dab, oder K oder Orn ist und deren Seitenkette mit der C-terminalen Aminosäure über eine Amidzyklisierung einen Ringschluss bildet,
oder bei R1 = NH2 die Position X12 E oder D ist und deren Seitenkette mit der N-terminalen Aminosäure über eine Amidzyklisierung einen Ringschluss bildet,
oder bei R1 = NH2 und R2 = COOH die N-terminale und die C-terminale Aminosäure des Peptids für einen Ringschluss über Amidbildung verwendet wird,
und gegebenenfalls vorhandene Lysine, die nicht für einen Ringschluss verwendet werden, über Spacer/Linker derivatisiert sein können.

4. Lineare sowie zyklische Peptide nach Anspruch 1 bis 3, **dadurch gekennzeichnet, dass** es sich um die folgenden Peptidsequenzen handelt,
R1--CAWHLGKLVWCT-R2
R1-DCAWHLGKLVWC--R2
R1--CAWHLGKLVWC--R2
R1-DCSWHLGKLVWCT-R2
R1-DCAWHHGKLVWCT-R2
R1-DCAWHQGKLVWCT-R2
R1-DCAWHGGKLVWCT-R2
R1-DCAWHGGKLVWCT-R2
R1-DCAWHGGHLVWCT-R2
R1-DCAWHGGKLVWCE-R2
wobei R1 = Amino-, Acetyl-
R2 = -COOH, -Amid, -GK, -GKK, -(βA)GK, -(βA)GKK, -(βA)(βA), -(βA)(βA)K ist,
R1-DCAWHLGKLVWCT-R2
R1-DCAWHQGKLVWCT-R2
R1-DCAYHLGKLVWCT-R2
R1-DCSWHLGKLVWCT-R2
R1-DCAYHQGKLVWCT-R2
R1-DCSWHQGKLVWCT-R2
R1-DCSYHLGKLVWCT-R2
R1-DCSYHQGKLVWCT-R2
R1--CAWHLGKLVWCT-R2
R1--CAWHQGKLVWCT-R2
R1--CAYHLGKLVWCT-R2
R1--CSWHLGKLVWCT-R2
R1--CAYHQGKLVWCT-R2
R1--CSWHQGKLVWCT-R2
R1--CSYHLGKLVWCT-R2
R1--CSYHQGKLVWCT-R2
R1-DCAWHLGKLVWC--R2
R1-DCAWHQGKLVWC--R2
R1-DCAYHLGKLVWC-R2
R1-DCSWHLGKLVWC--R2
R1-DCAYHQGKLVWC--R2
R1-DCSWHQGKLVWC--R2
R1-DCSYHLGKLVWC--R2
R1-DCSYHQGKLVWC--R2
R1--CAWHLGKLVWC--R2
R1--CAWHQGKLVWC--R2
R1--CAYHLGKLVWC--R2
R1--CSWHLGKLVWC--R2
R1--CAYHQGKLVWC--R2
R1--CSWHQGKLVWC--R2
R1--CSYH LGKLVWC--R2
R1--CSYHQGKLVWC--R2
wobei
R1 = Acetyl-
R2 = -COOH, -Amid ist,
gegebenenfalls die Cysteine in Position X02 und X12 für einen Ringschluss über Disulfidbrückenbildung verwendet werden,
oder die Cysteine in Position X02 und X12 durch Dpr oder Dab oder K oder Orn für X02 in Kombination mit D oder E für X12 ersetzt sind und für eine Amidzyklisierung über die Seitenketten verwendet werden,
oder die Cysteine in Position X02 und X12 durch D oder E für X02 in Kombination mit Dpr oder Dab oder K oder Orn für X12 ersetzt sind und für eine Amidzyklisierung über die Seitenketten verwendet werden.

5. Peptide nach einem der Ansprüche 1 bis 4, **dadurch gekennzeichnet, dass** die Peptide Immunglobuline oder Antigen-Immunglobulin-Komplexe in biologischen Flüssigkeiten binden.

6. Peptide nach einem der Ansprüche 1 bis 3, **dadurch gekennzeichnet, dass** der Linker/Spacer aus der Gruppe ausgewählt wird, bestehend aus:
- Carbonsäuren und deren Derivate
- Hydroxycarbonsäuren und deren Derivate
- Oligoalkoxyderivate und deren Oligomere
- α-Aminocarbonsäuren sowie deren Homo- und Heterooligomere
- α,ω-Aminocarbonsäuren sowie deren verzweigte Homo- und Heterooligomere
- sonstige Aminosäuren sowie deren lineare und verzweigte Homo- und Heterooligomere
- Amino-oligoalkoxy-alkylamine und deren lineare und verzweigte Homo- und Heterooligomere
- Maleinimidocarbonsäure und deren Derivate
- Oligomere von Alkylaminen
- 4-Alkylphenyl-Derivate
- 4-Oligoalkoxyphenyl- und 4-Oligoalkoxyphenoxy-Derivate
- Oligoalkylmercaptophenyl- und 4-Oligoalkylmercaptophenoxy-Derivate
- 4-Oligoalkylaminphenyl- und 4-Oligoalkylaminyphenoxy-Derivate
- (Oligoalkylbenzyl)-phenyl- bzw. 4-(Oligoalkylbenzyl)-phenoxy-Derivate sowie 4-(Oligoalkoxy-benzyl)-phenyl- bzw. 4-(Oligoalkoxybenzyl)-phenoxy-Derivate
- Trityl-Derivate
- Benzyloxyaryl- und Benzyloxyalkyl-Derivate
- Xanthen-3-yl-oxyalkyl-Derivate
- ω-(4-Alkylphenyl)- bzw. ω-(4-Alkylphenoxy)-alkansäure-Derivate
- Oligoalkyl-phenoxyalkyl- bzw. Oligoalkoxy-phenoxyalkyl-Derivate
- Carbamat-Derivate
- Amine
- Trialkylsilyl- und Dialkyl-alkoxysilyl-Derivate
- Alkyl- bzw. Aryl-Derivate
- Thiole und deren Derivate
- Thioether und deren Derivate
- Thiocarbonsäuren und deren Derivate
- Thiolcarbonsäuren und deren Derivate
- Sulfonsäuren und deren Derivate sowie
Kombinationen aus den aufgeführten Linkern oder Spacern.

7. Feste Phasen für die Affinitätschromatographie oder Festphasenextraktion aus organischen, anorganischen, synthetischen Polymeren oder aus Mischpolymeren, vorzugsweise quervernetzte Agarose, Cellulose, Kieselgel, Polyamid und Polyvinylalkohole, die gegebenenfalls chemisch aktiviert werden, mit an der Oberfläche der festen Phase immobilisierten Peptiden gemäss mindestens nach einem der Ansprüche 1 bis 6.

8. Feste Phasen nach Anspruch 7 wobei die Peptide kovalent oder durch Adsorption an die feste Trägerphase gebunden sind.

9. Feste Phasen nach Anspruch 7 oder 8, wobei die Peptide über K(Lys) an Position X08 kovalent an die feste Phase gebunden sind.

10. Feste Phasen nach Anspruch 8 oder 9 wobei die Peptide von der Trägeroberfläche über Linker/Spacer beabstandet sind.

11. Verfahren zur Adsorption von Immunglobulinen an einer festen Phase, wobei die Peptide nach Anspruch 1 bis 3 an eine übliche feste Phase für die Affinitätschromatographie oder an mobile feste Phasen gebunden werden, und die zu adsorbierenden immunglobulinhaltigen Proben mit den festen Phasen gemäss Anspruch 7 bis 10 in Kontakt gebracht werden.

12. Verfahren zur Adsorption von Immunglobulinen an einer festen Phase, wobei die Peptide nach Anspruch 1 bis 6 an eine übliche feste Phase für die Affinitätschromatographie oder an mobile feste Phasen gebunden werden, und die zu adsorbierenden immunglobulinhaltigen Proben mit Antikoagulantien behandeltes humanes Blutplasma oder humanes Vollblut sind und mit den festen Phasen gemäss Anspruch 7 bis 10 in Kontakt gebracht werden.

13. Verfahren nach Anspruch 12 zur Adsorption von Immunglobulinen aus immunglobulinhaltigen Proben, die Autoantikörper enthalten, die mit Autoimmunerkrankungen, vorzugsweise rheumatoide Erkrankungen, Multiple Sklerose, Myasthenia gravis sowie andere Autoantikörper-vermittelte Erkrankungen, assoziiert sind.

14. Vorrichtung zur Entfernung von Immunglobulinen aus immunglobulinhaltigen Proben an festen Phasen, wobei die Vorrichtung eine feste Phase gemäss einem der Ansprüche 7 bis 10 enthält und Einrichtungen vorgesehen sind zum Einlass von immunglobulinhaltigen Proben.

15. Verwendung von Peptiden nach einem der Ansprüche 1 bis 6, von festen Phasen nach einem der Ansprüche 7 bis 10 sowie Vorrichtungen nach Anspruch 14 zur Entfernung von Immunglobulinen aus immunglobulinhaltigen Proben.

## Claims

1. Peptides having the following amino acid sequence:
R¹-X01-X02-X03-X04-X05-X06-X07-X08-X09-X10-X11-X12-X13-R²,
wherein R¹, R² as well as X01 to X013 have the following meanings:
R¹ = amino, acetyl as well as spacer/linker, or deletion
X01 = A, D, E, G, deletion
X02 = C, S, D, E, diaminobutyric acid (Dab), diaminopropionic acid (Dpr), K, ornithine (Orn),
X03 = A, S, T
X04 = E, F, H, K, M, R, S, T, V, W, Y
X05 = H
X06 = D, H, K, L, M, N, Q, R
X07 = D, G
X08 = D, H, K, M, N, Q, R
X09 = K, L, R
X10 = V
X11 = W
X12 = C, S, D, E, diaminobutyric acid (Dab), diaminopropionic acid (Dpr), K, ornithine (Orn),
X13 = D, E, K, Q, R, S, T, deletion
R² = -COOH, -amide, -GK, -GKK, -(βA)GK, -(βA)GKK, -(βA)(βA), -(βA)(βA)K as well as spacer/linker, or deletion.

2. The peptide according to claim 1 in a linear as well as cyclic form, wherein the peptide ring closure is effected through disulfide bridging when two cysteines are present, or through amide cyclization, which is optionally effected through side chains, through the C and N termini or through a combination of these two possibilities.

3. The peptides according to claim 1 and/or 2, **characterized by** being:
a) the following peptide sequences:
R1-ACAWHLGKLVWCT-R2
R1-ECAWHLGKLVWCT-R2
R1-GCAWHLGKLVWCT-R2
R1--CAWHLGKLVWCT-R2
with a maximum of four simultaneous amino acid exchanges in the peptide positions as follows:
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, deletion
or
R1-DSAWHLGKLVWCT-R2
with a maximum of four simultaneous amino acid exchanges in the peptide positions as follows:
X01 = A, E, G, deletion
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, deletion
or
R1-DCSWHLGKLVWCT-R2
R1-DCTWHLGKLVWCT-R2
with a maximum of four simultaneous amino acid exchanges in the peptide positions as follows:
X01 = A, E, G, deletion
X02 = S
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, deletion,
or
R1-DCAEHLGKLVWCT-R2
R1-DCAFHLGKLVWCT-R2
Rl-DCAHHLGKLVWCT-R2
R1-DCAKHLGKLVWCT-R2
R1-DCAMHLGKLVWCT-R2
R1-DCARHLGKLVWCT-R2
R1-DCASHLGKLVWCT-R2
R1-DCATHLGKLVWCT-R2
R1-DCAVHLGKLVWCT-R2
R1-DCAWHLGKLVWCT-R2
R1-DCAYHLGKLVWCT-R2
with a maximum of four simultaneous amino acid exchanges in the peptide positions as follows:
X01 = A, E, G, deletion
X02 = S
X03 = S, T
X06 = G, H, K, M, N, Q, R
X07 = D
X08 = D, H, M, N, Q, R
X09 = K, R
X12 = S
X13 = D, E, K, Q, R, S, deletion,
or
R1-DCAWHDGKLVWCT-R2
R1-DCAWHEGKLVWCT-R2
R1-DCAWHGGKLVWCT-R2
R1-DCAWHHGKLVWCT-R2
R1-DCAWHKGKLVWCT-R2
R1-DCAWHMGKLVWCT-R2
R1-DCAWHNGKLVWCT-R2
R1-DCAWHQGKLVWCT-R2
R1-DCAWHRGKLVWCT-R2
with a maximum of four simultaneous amino acid exchanges in the peptide positions as follows:
X01 = A, E, G, deletion
X02 = S
X03 = S, T
X04 = E, F, H, K, M, R, S, T, V, Y
X07 = D
X08 = D, H, M, N, Q, R
X09 = K, R
X12 = S
X13 = D, E, K, Q, R, S, deletion,
or
R1-DCAWHLDKLVWCT-R2
with a maximum of four simultaneous amino acid exchanges in the peptide positions as follows:
X01 = A, E, G, deletion
X02 = S
X03 = S, T
X04 = E, F, H, K, M, R, S, T, V, Y
X06 = G, H, K, M, N, Q, R
X08 = D, H, M, N, Q, R
X09 = K, R
X12 = S
X13 = D, E, K, Q, R, S, deletion
or
R1-DCAWHLGDLVWCT-R2
R1-DCAWHLGHLVWCT-R2
R1-DCAWHLGKLVWCT-R2
R1-DCAWHLGMLVWCT-R2
R1-DCAWHLGNLVWCT-R2
R1-DCAWHLGQLVWCT-R2
R1-DCAWHLGRLVWCT-R2
with a maximum of four simultaneous amino acid exchanges in the peptide positions as follows:
X01 = A, E, G, deletion
X02 = S
X03 = S, T
X04 = E, F, H, K, M, R, S, T, V, Y
X06 = G, H, K, M, N, Q, R
X07 = D
X09 = K, R
X12 = S
X13 = D, E, K, Q, R, S, deletion,
or
R1-DCAWHLGKKVWCT-R2
R1-DCAWHLGKRVWCT-R2
with a maximum of four simultaneous amino acid exchanges in the peptide positions as follows:
X01 = A, E, G, deletion
X02 = S
X03 = S, T
X04 = E, F, H, K, M, R, S, T, V, Y
X06 = G, H, K, M, N, Q, R
X07 = D
X08 = D, H, M, N, Q, R
X12 = S
X13 = D, E, K, Q, R, S, deletion,
or
R1-DCAWHLGKLVWST-R2
with a maximum of four simultaneous amino acid exchanges in the peptide positions as follows:
X01 = A, E, G, deletion
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X13 = D, E, K, Q, R, S, deletion
or
R1-DCAWHLGKLVWCD-R2
R1-DCAWHLGKLVWCE-R2
R1-DCAWHLGKLVWCK-R2
R1-DCAWHLGKLVWCQ-R2
R1-DCAWHLGKLVWCR-R2
R1-DCAWHLGKLVWCS-R2
R1-DCAWHLGKLVWC--R2
with a maximum of four simultaneous amino acid exchanges in the peptide positions as follows:
X01 = A, E, G, deletion
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
wherein
R1 = amino, acetyl as well as spacer/linker,
R2 = -COOH, -amide, -GK, -GKK, -(βA)GK, -(βA)GKK, -(βA)(βA), -(βA)(βA)K as well as spacer/linker, the cysteines in positions X02 and X12 are optionally used for a ring closure through disulfide bridging, or the cysteines in positions X02 and X12 are replaced by Dpr or Dab or K or Orn for X02 in combination with D or E for X12 and employed for amide cyclization through the side chains;
or the cysteines in positions X02 and X12 are replaced by D or E for X02 in combination with Dpr or Dab or K or Orn for X12 and employed for amide cyclization through the side chains;
or, when R2 = COOH, the position X02 is Dpr or Dab or K or Orn and its side chain forms a ring closure with the C-terminal amino acid through amide cyclization;
or, when R1 = NH2, the position X12 is E or D and its side chain forms a ring closure with the N-terminal amino acid through amide cyclization;
or, when R1 = NH2 and R2 = COOH, the N-terminal and C-terminal amino acids of the peptide are used for ring closure through amide formation;
and any lysines which are not used for ring closure may be derivatized by spacers/linkers.

4. The linear as well as cyclic peptides according to claims 1 to 3, **characterized by** being the following peptide sequences:
R1--CAWHLGKLVWCT-R2
R1-DCAWHLGKLVWC--R2
R1--CAWH LGKLVWC--R2
R1-DCSWHLGKLVWCT-R2
R1-DCAWHHGKLVWCT-R2
R1-DCAWHQGKLVWCT-R2
R1-DCAWHGGKLVWCT-R2
R1-DCAWHGGKLVWCT-R2
R1-DCAWHGGHLVWCT-R2
R1-DCAWHGGKLVWCE-R2
wherein R1 = amino, acetyl
R2 = -COOH, -amide, -GK, -GKK, -(βA)GK, -(βA)GKK, -(βA)(βA), -(βA)(βA)K,
R1-DCAWHLGKLVWCT-R2
R1-DCAWHQGKLVWCT-R2
R1-DCAYHLGKLVWCT-R2
R1-DCSWHLGKLVWCT-R2
R1-DCAYHQGKLVWCT-R2
R1-DCSWHQGKLVWCT-R2
R1-DCSYHLGKLVWCT-R2
R1-DCSYHQGKLVWCT-R2
R1--CAWHLGKLVWCT-R2
R1--CAWHQGKLVWCT-R2
R1--CAYHLGKLVWCT-R2
R1-CSWHLGKLVWCT-R2
R1-CAYHQGKLVWCT-R2
R1-CSWHQGKLVWCT-R2
R1-CSYHLGKLVWCT-R2
R1-CSYHQGKLVWCT-R2
R1-DCAWHLGKLVWC-R2
R1-DCAWHQGKLVWC-R2
R1-DCAYHLGKLVWC-R2
R1-DCSWHLGKLVWC--R2
R1-DCAYHQGKLVWC--R2
R1-DCSWHQGKLVWC--R2
R1-DCSYHLGKLVWC--R2
R1-DCSYHQGKLVWC--R2
R1--CAWHLGKLVWC--R2
R1--CAWHQGKLVWC--R2
R1--CAYHLGKLVWC--R2
R1--CSWHLGKLVWC--R2
R1--CAYHQGKLVWC--R2
R1--CSWHQGKLVWC--R2
R1--CSYH LGKLVWC--R2
R1--CSYHQGKLVWC--R2
wherein
R1 = acetyl-
R2 = -COOH, -amide,
the cysteines in positions X02 and X12 are optionally used for ring closure through disulfide bridging;
or the cysteines in positions X02 and X12 are replaced by Dpr or Dab or K or Orn for X02 in combination with D or E for X12 and employed for amide cyclization through the side chains;
or the cysteines in positions X02 and X12 are replaced by D or E for X02 in combination with Dpr or Dab or K or Orn for X12 and employed for amide cyclization through the side chains.

5. The peptides according to any of claims 1 to 4, **characterized in that** said peptides bind immunoglobulins or antigen-immunoglobulin complexes in biological fluids.

6. The peptides according to any of claims 1 to 3, **characterized in that** said linker/spacer is selected from the group consisting of:
- carboxylic acids and their derivatives;
- hydroxycarboxylic acids and their derivatives;
- oligoalkoxy derivatives and their oligomers;
- α-aminocarboxylic acids and their homo- and heterooligomers;
- α,ω-aminocarboxylic acids and their branched homo- and heterooligomers;
- other amino acids and their linear and branched homo- and heterooligomers;
- amino-oligoalkoxy-alkylamines and their linear and branched homo- and heterooligomers;
- maleinimidocarboxylic acid and its derivatives;
- oligomers of alkylamines;
- 4-alkylphenyl derivatives;
- 4-oligoalkoxyphenyl and 4-oligoalkoxyphenoxy derivatives;
- oligoalkylmercaptophenyl and 4-oligoalkylmercaptophenoxy derivatives;
- 4-oligoalkylaminophenyl and 4-oligoalkylaminophenoxy derivatives;
- (oligoalkylbenzyl)phenyl and 4-(oligoalkylbenzyl)phenoxy derivatives;
- and 4-(oligoalkoxybenzyl)phenyl and 4-(oligoalkoxybenzyl)phenoxy derivatives;
- trityl derivatives;
- benzyloxyaryl and benzyloxyalkyl derivatives;
- xanthen-3-yloxyalkyl derivatives;
- ω-(4-alkylphenyl) and ω-(4-alkylphenoxy)alkanoic acid derivatives;
- oligoalkylphenoxyalkyl and oligoalkoxyphenoxyalkyl derivatives;
- carbamate derivatives;
- amines;
- trialkylsilyl and dialkylalkoxysilyl derivatives;
- alkyl and aryl derivatives;
- thiols and their derivatives;
- thioethers and their derivatives;
- thiocarboxylic acids and their derivatives;
- thiolcarboxylic acids and their derivatives;
- sulfonic acids and their derivatives; and
- combinations of the mentioned linkers or spacers.

7. Solid phases for affinity chromatography or solid-phase extraction consisting of organic, inorganic, synthetic polymers or of mixed polymers, preferably cross-linked agarose, cellulose, silica gel, polyamide and polyvinyl alcohols, which are optionally chemically activated, with peptides according to at least one of claims 1 to 6 immobilized on the surface of the solid phase.

8. The solid phases according to claim 7, wherein the peptides are bound to the solid support phase covalently or by adsorption.

9. The solid phases according to claim 7 or 8, wherein the peptides are covalently bound to the solid phase through K(Lys) on position X08.

10. The solid phases according to claim 8 or 9, wherein the peptides are distanced from the support surface by linkers/spacers.

11. A method for the adsorption of immunoglobulins to a solid phase, wherein the peptides according to claims 1 to 3 are bound to a solid phase usual in affinity chromatography or to mobile solid phases, and the immunoglobulin-containing samples to be adsorbed are contacted with the solid phases according to claims 7 to 10.

12. A method for the adsorption of immunoglobulins to a solid phase, wherein the peptides according to claims 1 to 6 are bound to a solid phase usual in affinity chromatography or to mobile solid phases, and the immunoglobulin-containing samples to be adsorbed are anticoagulant-treated human blood plasma or human whole blood and are contacted with the solid phases according to claims 7 to 10.

13. The method according to claim 12 for the adsorption of immunoglobulins from immunoglobulin-containing samples which contain auto-antibodies related to autoimmune diseases, preferably rheumatoid diseases, multiple sclerosis, myasthenia gravis and other auto-antibody-mediated diseases.

14. A device for removing immunoglobulins from immunoglobulin-containing samples on solid phases, wherein the device contains a solid phase according to any of claims 7 to 10, and means for the entry of immunoglobulin-containing samples are provided.

15. Use of peptides according to any of claims 1 to 6, of solid phases according to any of claims 7 to 10 and of devices according to claim 14 for removing immunoglobulins from immunoglobulin-containing samples.

## Revendications

1. Peptides ayant la séquence d'acides aminés suivante,
**R¹-X01-X02-X03-X04-X05-X06-X07-X08-X09-X10-X11-X12-X13-R²**,
où R¹, R² ainsi que X01 à X013 ont la signification suivante :
R¹ = amino-, acétyl- ainsi qu'un espaceur/linker, ou une délétion
X01 = A, D, E, G, une délétion
X02 = C, S, D, E, l'acide diaminobutyrique (Dab), l'acide diaminopropionique (Dpr), K, l'ornithine (Orn),
X03 = A, S, T
X04 = E, F, H, K, M, R, S, T, V, W, Y
X05 = H
X06 = E, G, H, K, L, M, N, Q, R
X07 = D, G
X08 = D, H, K, M, N, Q, R
X09 = K, L, R
X10 = V
X11 = W
X12 = C, S, D, E, l'acide diaminobutyrique (Dab), l'acide diaminopropionique (Dpr), K, l'ornithine (Orn),
X13 = D, E, K, Q, R, S, T, une délétion
R² = -COOH, -amide, -GK, -GKK, -(βA) GK, -(βA) GKK, -(βA)(βA), -(βA)(βA)K ainsi qu'un espaceur/linker, ou une délétion.

2. Peptide selon la revendication 1 sous forme linéaire ainsi que cyclique, dans lequel la cyclisation du peptide en présence de deux cystéines, a lieu par la formation de ponts disulfure ou une cyclisation d'amide, qui a éventuellement lieu au niveau des chaînes latérales, au niveau de l'extrémité C-terminale et de l'extrémité N-terminale ou par une combinaison des deux possibilités.

3. Peptides selon la revendication 1 et/ou 2, **caractérisés en ce qu'**il s'agit
a) des séquences peptidiques suivantes,
R1-ACAWHLGKLVWCT-R2
R1-ECAWHLGKLVWCT-R2
R1-GCAWHLGKLVWCT-R2
R1--CAWHLGKLVWCT-R2
avec au maximum quatre substitutions simultanées d'acides aminés dans les positions de peptides comme suit,
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, une délétion
ou
R1-DSAWHLGKLVWCT-R2
avec au maximum quatre substitutions simultanées d'acides aminés dans les positions de peptides comme suit,
X01 = A, E, G, une délétion
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, une délétion
ou
R1-DCSWHLGKLVWCT-R2
R1-DCTWHLGKLVWCT-R2
avec au maximum quatre substitutions simultanées d'acides aminés dans les positions de peptides comme suit,
X01 = A, E, G, une délétion
X02 = S
X04 = E, F, H, K, M, R, S, T, V, Y
X06 = G, H, K, M, N, Q, R
X07 = D
X08 = D, H, M, N, Q, R
X09 = K, R
X12 = S
X13 = D, E, K, Q, R, S, une délétion,
ou
R1-DCAEHLGKLVWCT-R2
R1-DCAFHLGKLVWCT-R2
R1-DCAHHLGKLVWCT-R2
R1-DCAKHLGKLVWCT-R2
R1-DCAMHLGKLVWCT-R2
R1-DCARHLGKLVWCT-R2
R1-DCASHLGKLVWCT-R2
R1-DCATHLGKLVWCT-R2
R1-DCAVHLGKLVWCT-R2
R1-DCAWHLGKLVWCT-R2
R1-DCAYHLGKLVWCT-R2
avec au maximum quatre substitutions simultanées d'acides aminés dans les positions de peptides comme suit,
X01 = A, E, G, une délétion
X02 = S
X03 = S, T
X06 = G, H, K, M, N, Q, R
X07 = D
X08 = D, H, M, N, Q, R
X09 = K, R
X12 = S
X13 = D, E, K, Q, R, S, une délétion,
ou
R1-DCAWHDGKLVWCT-R2
R1-DCAWHEGKLVWCT-R2
R1-DCAWHGGKLVWCT-R2
R1-DCAWHHGKLVWCT-R2
R1-DCAWHKGKLVWCT-R2
R1-DCAWHMGKLVWCT-R2
R1-DCAWHNGKLVWCT-R2
R1-DCAWHQGKLVWCT-R2
R1-DCAWHRGKLVWCT-R2
avec au maximum quatre substitutions simultanées d'acides aminés dans les positions de peptides comme suit,
X01 = A, E, G, une délétion
X02 = S
X03 = S, T
X04 = E, F, H, K, M, R, S, T, V, Y
X07 = D
X08 = D, H, M, N, Q, R
X09 = K, R
X12 = S
X13 = D, E, K, Q, R, S, une délétion,
ou
R1-DCAWHLDKLVWCT-R2
avec au maximum quatre substitutions simultanées d'acides aminés dans les positions de peptides comme suit,
X01 = A, E, G, une délétion
X02 = S
X03 = S, T
X04 = E, F, H, K, M, R, S, T, V, Y
X06 = G, H, K, M, N, Q, R
X08 = D, H, M, N, Q, R
X09 = K, R
X12 = S
X13 = D, E, K, Q, R, S, une délétion
ou
R1-DCAWHLGDLVWCT-R2
R1-DCAWHLGHLVWCT-R2
R1-DCAWHLGKLVWCT-R2
R1-DCAWHLGMLVWCT-R2
R1-DCAWHLGNLVWCT-R2
R1-DCAWHLGQLVWCT-R2
R1-DCAWHLGRLVWCT-R2
avec au maximum quatre substitutions simultanées
d'acides aminés dans les positions de peptides comme suit,
X01 = A, E, G, une délétion
X02 = S
X03 = S, T
X04 = E, F, H, K, M, R, S, T, V, Y
X06 = G, H, K, M, N, Q, R
X07 = D
X09 = K, R
X12 = S
X13 = D, E, K, Q, R, S, une délétion,
ou
R1-DCAWHLGKKVWCT-R2
R1-DCANHLGKRVWCT-R2
avec au maximum quatre substitutions simultanées d'acides aminés dans les positions de peptides comme suit,
X01 = A, E, G, une délétion
X02 = S
X03 = S, T
X04 = E, F, H, K, M, R, S, T, V, Y
X06 = G, H, K, M, N, Q, R
X07 = D
X08 = D, H, M, N, Q, R
X12 = S
X13 = D, E, K, Q, R, S, une délétion,
ou
R1-DCAWHLGKLVWST-R2
avec au maximum quatre substitutions simultanées
d'acides aminés dans les positions de peptides comme suit,
X01 = A, E, G, une délétion
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X13 = D, E, K, Q, R, S, une délétion
ou
R1-DCAWHLGKLVWCD-R2
R1-DCAWHLGKLVWCE-R2
R1-DCAWHLGKLVWCK-R2
R1-DCAWHLGKLVWCQ-R2
R1-DCAWHLGKLVWCR-R2
R1-DCAWHLGKLVWCS-R2
R1-DCAWHLGKLVWC--R2
avec au maximum quatre substitutions simultanées
d'acides aminés dans les positions de peptides comme suit,
X01 = A, E, G, une délétion
X02 = S
X03 = S, T
X04 = E, F, H, K, M, R, S, T, V, Y
X06 = G, H, K, M, N, Q, R
X07 = D
X08 = D, H, M, N, Q, R
X09 = K, R
X12 = S
où,
R1 = amino-, acétyl- ainsi qu'un espaceur/linker,
R2 = -COOH, -amide, -GK, -GKK, -(βA)GK, -(βA)GKK, -(βA) (βA), -(βA) (βA) K ainsi qu'un espaceur/linker, éventuellement les cystéines en position X02 et X12 sont utilisées pour une cyclisation par formation de ponts disulfure,
ou les cystéines en position X02 et X12 sont substituées par Dpr ou Dab ou K ou Orn pour X02 en combinaison avec D ou E pour X12 et sont utilisées pour une cyclisation d'amide par l'intermédiaire des chaînes latérales,
ou les cystéines en position X02 et X12 sont substituées par D ou E pour X02 en combinaison avec Dpr ou Dab ou K ou Orn pour X12 et sont utilisées pour une cyclisation d'amide par l'intermédiaire des chaînes latérales,
ou, si R2 = COOH, la position X02 est Dpr ou Dab, ou K ou Orn, et sa chaîne latérale, avec l'acide aminé C-terminal, forme une cyclisation par l'intermédiaire d'une cyclisation d'amide,
ou, si R1 = NH2, la position X12 est E ou D, et sa chaîne latérale, avec l'acide aminé N-terminal, forme une cyclisation par l'intermédiaire d'une cyclisation d'amide
ou, si R1 = NH2 et R2 = COOH, l'acide aminé N-terminal et C-terminal du peptide est utilisé pour une cyclisation par formation d'amide,
et les lysines éventuellement présentes qui ne sont pas utilisées pour une cyclisation peuvent être dérivées sur un espaceur/linker.

4. Peptides linéaires ainsi que cycliques selon les revendications 1 à 3, **caractérisés en ce qu'**il s'agit des séquences peptidiques suivantes,
R1--CAWHLGKLVWCT-R2
R1-DCAWHLGKLVWC--R2
R1--CAWHLGKLVWC--R2
R1-DCSWHLGKLVWCT-R2
R1-DCAWHHGKLVWCT-R2
R1-DCAWHQGKLVWCT-R2
R1-DCAWHGGKLVWCT-R2
R1-DCAWHGGKLVWCT-R2
R1-DCAWHGGHLVWCT-R2
R1-DCAWHGGKLVWCE-R2
où
R1 = amino-, acétyl-
R2 = -COOH, -amide, -GK, -GKK, -(βA)GK, -(βA)GKK, -(βA)(βA), -(βA)(βA)K,
R1-DCAWHLGKLVWCT-R2
R1-DCAWHQGKLVMCT-R2
R1-DCAYHLGKLVWCT-R2
R1-DCSWHLGKLVWCT-R2
R1-DCAYHQGKLVWCT-R2
R1-DCSWHQGKLVWCT-R2
R1-DCSYHLGKLVWCT-R2
R1-DCSYHQGKLVWCT-R2
R1--CAWHLGKLVWCT-R2
R1--CAWHQGKLVWCT-R2
R1--CAYHLGKLVWCT-R2
R1--CSWHLGKLVWCT-R2
R1--CAYHQGKLVWCT-R2
R1--CSWHQGKLVWCT-R2
R1--CSYHLGKLVWCT-R2
R1--CSYHQGKLVWCT-R2
R1-DCAWHLGKLVWC--R2
R1-DCAWHQGKLVWC--R2
R1-DCAYHLGKLVWC--R2
R1-DCSWHLGKLVWC--R2
R1-DCAYHQGKLVWC--R2
R1-DCSWHQGKLVWC--R2
R1-DCSYHLGKLVWC--R2
R1-DCSYHQGKLVWC--R2
R1--CAWHLGKLVWC--R2
R1--CAWHQGKLVWC--R2
R1--CAYHLGKLVWC--R2
R1--CSWHLGKLVWC--R2
R1--CAYHQGKLVWC--R2
R1--CSWHQGKLVWC--R2
R1--CSYHLGKLVWC--R2
R1--CSYHQGKLVWC--R2
où
R1 = acétyl-
R2 = -COOH, -amide,
éventuellement les cystéines en position X02 et X12 sont utilisées pour une cyclisation par formation de ponts disulfure,
ou les cystéines en position X02 et X12 sont substituées par Dpr ou Dab ou K ou Orn pour X02 en combinaison avec D ou E pour X12 et sont utilisées pour une cyclisation d'amide par l'intermédiaire des chaînes latérales,
ou les cystéines en position X02 et X12 sont substituées par D ou E pour X02 en combinaison avec Dpr
ou Dab ou K ou Orn pour X12 et sont utilisées pour une cyclisation d'amide par l'intermédiaire des chaînes latérales.

5. Peptides selon l'une quelconque des revendications 1 à 4, **caractérisés en ce que** les peptides lient des immunoglobulines ou des complexes antigène-immunoglobuline dans des fluides biologiques.

6. Peptides selon l'une quelconque des revendications 1 à 3, **caractérisés en ce que** l'linker/espaceur est choisi dans le groupe constitué par :
- les acides carboxyliques et leurs dérivés
- les acides hydroxycarboxyliques et leurs dérivés
- les dérivés d'oligoalcoxy et leurs oligomères
- les acides α-aminocarboxyliques ainsi que leurs homo- et hétéro-oligomères
- les acides α,ω-aminocarboxyliques ainsi que leurs homo- et hétéro-oligomères ramifiés
- d'autres acides aminés ainsi que leurs homo- et hétéro-oligomères linéaires et ramifiés
- les amino-oligoalcoxy-alkylamines et leurs homo- et hétéro-oligomères linéaires et ramifiés
- les acides maléinimidocarboxyliques et leurs dérivés
- les oligomères d'alkylamines
- les dérivés de 4-alkylphényle
- les dérivés de 4-oligoalcoxyphényle et de 4-oligoalcoxyphénoxy
- les dérivés d'oligoalkylmercaptophényle et de 4-oligoalkylmercaptophénoxy
- les dérivés de 4-oligoalkylaminophényle et de 4-oligoalkylaminophénoxy
- les dérivés d'(oligoalkylbenzyl)-phényle ou de 4-(oligoalkylbenzyl)-phénoxy ainsi que les dérivés de 4-(oligoalcoxybenzyl)-phényle ou de 4-(oligoalcoxybenzyl)-phénoxy
- les dérivés de trityle
- les dérivés de benzyloxyaryle et de benzyloxyalkyle
- les dérivés de xanthén-3-yl-oxyalkyle
- les dérivés d'acide ω-(4-alkylphényl)- ou ω-(4-alkylphénoxy)-alcanoïque
- les dérivés d'oligoalkyl-phénoxyalkyle ou d'oligoalcoxy-phénoxyalkyle
- les dérivés de carbamate
- les amines
- les dérivés de trialkylsilyle et de dialkylalcoxysilyle
- les dérivés d'alkyle ou d'aryle
- les thiols et leurs dérivés
- les thioéthers et leurs dérivés
- les acides thiocarboxyliques et leurs dérivés
- les acides thiolcarboxyliques et leurs dérivés
- les acides sulfoniques et leurs dérivés, ainsi que
- des combinaisons des linkers ou espaceurs mentionnés.

7. Phases solides pour la chromatographie d'affinité ou l'extraction en phase solide à partir de polymères ou de mélanges de polymères organiques, inorganiques, synthétiques, de préférence d'agarose réticulée, de cellulose, de gel de silice, de polyamide et de poly(alcool de vinyle), qui sont éventuellement chimiquement activés, avec, à la surface de la phase solide, des peptides immobilisés selon au moins l'une quelconque des revendications 1 à 6.

8. Phases solides selon la revendication 7, dans lesquelles les peptides sont liés à la phase support solide de façon covalente ou par adsorption.

9. Phases solides selon la revendication 7 ou 8, dans lesquelles les peptides sont liés à la phase solide de façon covalente par l'intermédiaire de K(Lys) en position X08.

10. Phases solides selon la revendication 8 ou 9, dans lesquelles les peptides sont éloignés de la surface de support par des linkers/espaceurs.

11. Procédé d'adsorption d'immunoglobulines sur une phase solide, dans lequel les peptides selon les revendications 1 à 3 sont liés à une phase solide habituelle pour la chromatographie d'affinité ou à des phases solides mobiles, et les échantillons contenant les immunoglobulines à adsorber sont mis en contact avec les phases solides selon les revendications 7 à 10.

12. Procédé d'adsorption d'immunoglobulines sur une phase solide, dans lequel les peptides selon les revendications 1 à 6 sont liés à une phase solide habituelle pour la chromatographie d'affinité ou à des phases solides mobiles, et les échantillons contenant les immunoglobulines à adsorber sont du plasma sanguin humain traité avec des anticoagulants ou du sang total humain et sont mis en contact avec les phases solides selon les revendications 7 à 10.

13. Procédé selon la revendication 12 d'adsorption d'immunoglobulines à partir d'échantillons contenant des immunoglobulines, qui contiennent des auto-anticorps associés à des maladies auto-immunes, de préférence à des maladies rhumatoïdes, à la sclérose en plaques, à la myasthénie grave ainsi qu'à d'autres maladies provoquées par des auto-anticorps.

14. Dispositif d'élimination d'immunoglobulines dans des échantillons contenant des immunoglobulines sur des phases solides, dans lequel le dispositif contient une phase solide selon l'une quelconque des revendications 7 à 10 et des appareils sont prévus pour l'admission des échantillons contenant des immunoglobulines.

15. Utilisation de peptides selon l'une quelconque des revendications 1 à 6, de phases solides selon l'une quelconque des revendications 7 à 10 ainsi que de dispositifs selon la revendication 14 pour éliminer des immunoglobulines dans des échantillons contenant des immunoglobulines.
